# EUROPÄISCHE PATENTSCHRIFT

(11) **EP 3 496 818 B1**
(45) Veröffentlichungstag und Bekanntmachung des Hinweises auf die Patenterteilung: **20.01.2021**
(21) Anmeldenummer: 17731551.2
(22) Anmeldetag: 20.06.2017
(51) Int. Cl.: A61Q 5/10, A61K 8/06, A61K 8/14, A61K 8/34, A61K 8/39, A61K 8/46, A61K 8/86, A61K 8/37, A61K 8/38, A61K 8/49, A61Q 5/08

(54) **VERFAHREN ZUR HERSTELLUNG VON HAARFÄRBEMITTELN MIT UNILAMELLAREN UND MULTILAMELLAREN VESIKELN**
PROCESS FOR PREPARING HAIR DYE AGENTS HAVING UNILAMELLAR AND MULTILAMELLAR VESICLES
PROCÉDÉ DE PRÉPARATION DES AGENTS DE COLORATION À VÉSICULES UNILAMELLAIRES ET MULTILAMELLAIRES

(30) Priorität: 09.08.2016 DE 102016214716
(43) Veröffentlichungstag der Anmeldung: 19.06.2019
(73) Patentinhaber: Henkel AG & Co. KGaA, 40589 Düsseldorf (DE)
(72) Erfinder: MÜLLER, Burkhard, 40221 Düsseldorf (DE); BONNIN, Lucile, 40215 Düsseldorf (DE)
(86) Internationale Anmeldenummer: PCT/EP2017/065084
(87) Internationale Veröffentlichungsnummer: WO 2018/028861

(56) Entgegenhaltungen:
- EP-A1- 1 433 475
- EP-B1- 1 761 235
- WO-A1-2015/112787
- DE-A1-102005 053 349
- DE-A1-102011 085 754
- DE-A1-102013 215 583
- DE-A1-102014 226 321
- DE-A1-102015 222 985
- DE-C1- 19 719 504

## Beschreibung

Die vorliegende Erfindung liegt auf dem Gebiet der Kosmetik. Gegenstand der vorliegenden Erfindung ist ein Verfahren zur Herstellung von Mitteln (A) zur Farbveränderung von keratinischen Fasern, welche in einem wässrigen Medium unilamellare Vesikel (ULV) und/oder multilamellare Vesikel (MLV) umfassen und weiterhin mindestens einen Fettalkohol, mindestens einen ethoxylierten Fettalkohol, mindestens ein anionisches Tensid und bestimmte Mengenbereiche an farbverändernden Verbindungen in Salzform enthalten.

Die Anwendung von Emulsionen ist in der Kosmetik weit verbreitet. In einer Emulsion liegt ein fein verteiltes Gemisch zweier Flüssigkeiten, wie beispielsweise Fettkörper (Öle, Fettalkohole, Kohlenwasserstoffe oder auch Fettsäuretriglyceride) und Wasser vor. Eine Theorie zu Emulsionen ist, dass eine der Flüssigkeiten (Phase) kleine Tröpfchen bildet, die verteilt in der anderen Flüssigkeit (Phase) vorliegen. Die Phase, welche die Tröpfchen bildet, wird als innere Phase oder auch disperse Phase bezeichnet. Die Phase, in der die Tröpfchen schwimmen, wird die äußere Phase oder auch die kontinuierliche Phase genannt.

Bei Emulsionen, die eine Wasserphase und eine Ölphase umfassen, werden Öl-in-Wasser Emulsionen (O/W-Emulsionen) und Wasser-in-ÖI-Emulsionen (W/O-Emulsionen) unterschieden. Klassische O/W-Emulsionen werden in der Literatur oft als Öltröpfchen beschrieben, die in der kontinuierlichen Wasserphase dispergiert sind und an der Grenzfläche beider Phasen durch Tenside oder Emulgatoren stabilisiert sind. Letztere formen einen Film um die Öltröpfchen und sind so in der Lage, die Oberflächenspannung herabzusetzen. In komplexen kosmetischen Formulierungen wird jedoch in der Regel eine Vielzahl verschiedener Inhaltstoffe eingesetzt, wodurch kompliziertere mehrphasige Systeme entstehen.

Im Bereich der Haarfärbe- und Blondier-Mittel ist der Einsatz von Formulierungen bekannt, die auf hohen Mengen an Fettalkoholen (Fett-Amphiphilen) und Tensiden basieren. In Formulierungen dieses Typs entstehen oft lamellare Netzwerke, die auch als lamellare Gelnetzwerke oder multilamellare Strukturen beschrieben werden.

WO 2015/112787 A1 beschreibt Haarfärbemittel, welche eine Multi-lamellare Emulsion umfassen und welche neben farbgebenden Substanzen die Kombination aus polaren nichtionischen Tensiden und weniger polaren nichtionischen Tensiden enthalten.

In EP 1 433 475 A1 werden Haarfärbemittel adressiert, die neben Ammoniak und Oxidationsfarbstoffen mindestens 10 Gew.-% einer mesomorphen Phase enthalten, wobei der Begriff mesomorphe Phasen auch lamellare Phasen umfasst.

EP 1 761 235 B1 schließlich beansprucht Färbemittel, welche die Oxidationsfarbstoffvorprodukte eingekapselt in oder adsorbiert an multilamellaren Vesikeln enthalten.

DE 10 2015 222 985 A1 betrifft Färbemittel für die oxidative Farbveränderung von Keratinfasern, welche ein Abyssinian Öl enthalten und welche eine verbesserte Waschechtheit der mit dem Färbemittel gefärbten Fasern bewirken.

Die in diesen Dokumenten beschriebenen Färbemittel können jedoch im Hinblick auf ihre anwendungstechnischen Eigenschaften, insbesondere im Hinblick auf ihre Stabilität noch nicht als optimal betrachtet werden.

Zur Erzeugung von permanenten, intensiven Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch intensive, hervorragende, lang anhaltende Färbeergebnisse aus. Für natürlich wirkende Färbungen kann eine Mischung aus einer größeren Zahl von Oxidationsfarbstoffvorprodukten eingesetzt werden, wobei in vielen Fällen weiterhin zusätzlich direktziehende Farbstoffe zur Nuancierung verwendet werden.

Oxidationsfarbstoffvorprodukte vom Entwicklertyp basieren typischerweise auf der Grundstruktur des p-Phenylendiamins, des p-Aminophenols oder von heterozyklischen Di- oder Poly-Aminoverbindungen. Substanzen dieses Typs sind gegenüber Luftsauerstoff höchst empfindlich und werden zur Stabilisierung meist in Form ihrer physiologisch verträglichen Salze eingesetzt, d.h. die in den Substanzen vorhandenen Aminogruppen werden - ganz oder teilweise - in Ammoniumgruppen überführt und durch Gegenionen (Chloride, Bromide, Hydrogensulfate oder auch Sulfate) neutralisiert. Wünscht sich der Anwender die Färbung seiner Haare in einem besonders dunklen Farbton, wie beispielsweise einer dunkelbraunen oder schwarzen Nuance, so greift er zu einem entsprechenden Färbemittel mit besonders hohem Farbstoffgehalt. Bedingt durch den hohen Gehalt an Oxidationsfarbstoffvorprodukten ist auch der entsprechende Salzgehalt in diesen Mitteln sehr hoch.

Emulsionen wie beispielsweise O/W-Emulsionen reagieren gegenüber einer Erhöhung ihres Salzgehaltes oft sehr empfindlich. Die Gefahr, dass eine Emulsion bzw. ein Färbemittel sich trennt und als nicht lagerstabil erweist, ist bei Nuancen mit hohem Farbstoffgehalt daher besonders groß.

Eine erste Aufgabe der vorliegenden Erfindung lag daher in der Bereitstellung von Färbemitteln mit verbesserter Salztoleranz. Zudem sollte auch die Lagerstabilität der Färbemittel mit hohem Salzgehalt verbessert werden.

Die grundlegende Voraussetzung hierbei war, dass die Färbemittel die oben genannten Verbesserungen im Hinblick auf ihre Salztoleranz aufzeigen sollten, ohne Einbußen im Hinblick auf die weiteren anwendungstechnischen Eigenschaften, zu erleiden. Die Farbintensitäten, Waschechtheiten und Lichtechtheiten dieser Mittel sollten sich gegenüber den aus dem Stand der Technik bekannten Mitteln also nicht verschlechtern und optimaler Weise weiter verbessern.

Überraschenderweise hat sich nun herausgestellt, dass farbverändernde Salze in einer Gesamtmenge von bis zu 6,0 Gew.-% über einen langen Zeitraum stabil in ein Färbemittel eingearbeitet werden können, wenn dieses Färbemittel in einem wässrigen Medium uni- und/oder multi-lamellare Vesikel umfasst, und wenn das lamellare System, in welchem sich die Vesikel befinden, zusätzlich Fettalkohole, ethoxylierte Fettalkohole einer Formel (I) und anionische Tenside einer Formel (II) enthält. Trotz des hohen Salzgehaltes erwiesen sich diese vesikelhaltigen Systeme als besonders lagerstabil, und bei ihrer Anwendung konnten intensive Färbungen mit guten Echtheitseigenschaften erhalten werden. Auch die Stabilität dieser Vesikelsysteme gegenüber Temperaturschwankungen war ausgezeichnet.

Ein erster Gegenstand der vorliegenden Erfindung ist ein Verfahren zur Herstellung eines Mittels (A) zur Farbveränderung von keratinischen Fasern, insbesondere menschlichen Haaren, welches in einem wässrigen Medium unilamellare Vesikel (ULV) und/oder multilamellare Vesikel (MLV) umfasst, wobei das Mittel enthält
(a1) mindestens einen C₈-C₃₀-Fettalkohol
(a2) mindestens einen ethoxylierten Fettalkohol der Formel (I), 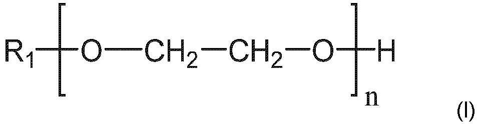 worin
   R1 für eine lineare oder verzweigte, gesättigte oder ungesättigte C₈-C₃₀-Alkylgruppe steht und,
   n für eine ganze Zahl von 10 bis 100 steht,
(a3) mindestens ein anionisches Tensid der Formel (II) 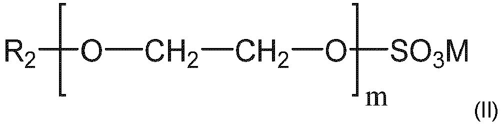 worin
   R2 für eine lineare oder verzweigte, gesättigte oder ungesättigte C₈-C₃₀-Alkylgruppe steht,
   m für eine ganze Zahl von 0 bis 10 steht, und
   M für ein Wasserstoffatom, ein Äquivalent eines Natriumions, eines Kaliumions oder eines Ammoniumions (NH₄⁺) steht, und
(a4) ein oder mehrere farbverändernde Salze aus der Gruppe der Sulfate, der Hydrogensulfate, der Chloride und/oder der Bromide in einer Gesamtmenge von 0,9 bis 3,4 Gew.-%, wobei die Gesamtmenge auf das Gesamtgewicht des Mittels (A) bezogen ist, dadurch gekennzeichnet, dass das Verfahren die folgenden Schritte umfasst
   (I) Erhitzen eines Gemisches aus (a1) C₁₂-C₃₀-Fettalkohol, (a2) ethoxyliertem Fettalkohol der Formel (I), (a3) anionischem Tensid der Formel (II) und einer ersten Teilmenge Wasser auf eine Temperatur von 65 bis 85 °C in einem ersten Behältnis,
   (II) Herstellung eines Gemisches aus einer zweiten Teilmenge Wasser und den farbverändernden Salzen aus der Gruppe der Sulfate, der Hydrogensulfate, der Chloride (a4) in einem zweiten Behältnis,
   (III) Zugabe der in Schritt (II) erhaltenen Zubereitung zu der in Schritt (I) erhaltenen, auf 40 bis 60 °C temperierten Zubereitung unter konstantem Rühren mit einem Propeller-Rührwerk bei mindestens 500 Umdrehungen pro Minute,
   (IV) Abkühlen des in Schritt (III) erhaltenen Gemisches unter ständigem Rühren auf 20 bis 40 °C.

### Keratinische Fasern

Bei dem Mittel (A) handelt es sich um ein Mittel zur Farbveränderung von keratinischen Fasern, insbesondere menschlichen Haaren. Besonders bevorzugt wird das Mittel (A) zum oxidativen Färben von keratinischen Fasern, insbesondere menschlichen Haaren, eingesetzt.

Unter keratinischen Fasern sind Wolle, Pelze, Federn und insbesondere menschliche Haare zu verstehen. Die erfindungsgemäßen Mittel zur oxidativen Farbveränderung können prinzipiell aber auch zur Farbveränderung anderer Naturfasern, wie z. B. Baumwolle, Jute, Sisal, Leinen oder Seide, modifizierter Naturfasern, wie beispielsweise Regeneratcellulose, Nitro-, Alkyl- oder Hydroxyalkyl- oder Acetylcellulose verwendet werden.

### Unilamellare und/oder multilamellare Vesikel

Die durch das erfindungsgemäße Verfahren hergestellten Mittel umfassen oder enthalten in einem wässrigen Medium unilamellare Vesikel (ULV) und/oder multilamellare Vesikel (MLV).

Vesikel sind sphärisch lamellare, polydisperse amphiphile Strukturen von Vesikelbildnern, die sich in mindestens einer Doppelschicht anordnen. Als erfindungsgemäße Vesikelbildner enthalten diese Doppelschichten Fettalkohole (a1), bestimmte nichtionische Tenside (a2) und bestimmte anionische Tenside (a3).

Vesikel können als unilamellare Vesikel (ULV) und multilamellare Vesikel (MLV) vorliegen.

Bei unilamellaren Vesikeln umhüllt eine aus Vesikelbildnern (z.B. Fettalkohole und Tenside) gebildete Doppelschicht einen hydrophilen, wasserhaltigen Innenraum. Multilamellare Vesikel bestehen üblicherweise aus mehreren, einander sphärisch umschließenden Vesikeln, wobei die Doppelmembranen dieser sich umschließenden Vesikel wie Zwiebelschalen angeordnet sind.

Unilamellare Vesikel umfassen eine einzige sphärische Doppelmembran und werden, je nach Durchmesser, als SULV (small unilamelar vesicle, maximaler Durchmesser 50 nm (Nanometer)) oder LULV (large unilamellar vesicle, Durchmesser größer 50 nm (Nanometer)) bezeichnet. Der Durchmesser von multilamellaren Vesikeln ist in der Regel größer und liegt im Bereich von 100 nm (Nanometer) bis zu einigen Mikrometern.

Die Wirkstoffe des kosmetischen Färbemittels (insbesondere die in Salzform vorliegenden Oxidationsfarbstoffvorprodukte und/oder die direktziehenden Farbstoffe) können sich innerhalb des wasserhaltigen Innenraums des Vesikels oder auch außerhalb des Vesikels befinden und stehen in diesem Falle gegebenenfalls mit den an der Oberfläche der Vesikelmembran absorbierten Wirkstoffen im Gleichgewicht.

Die durch das erfindungsgemäße Verfahren hergestellten Mittel umfassen oder enthalten in einem wässrigen Medium unilamellare Vesikel (ULV) und/oder multilamellare Vesikel (MLV). Mittel, welche multilamellare Vesikel (MLV) enthalten, haben sich als ganz besonders stabil erwiesen.

In einer besonders bevorzugten Ausführungsform ist ein durch das erfindungsgemäße Verfahren hergestelltes Mittel (A) dadurch gekennzeichnet, dass es multilamellare Vesikel (MLV) umfasst.

### Polarisationsmikroskopie

Die Anwesenheit von Vesikeln kann mittels Polarisationsmikroskopie detektiert werden. Ein Polarisationsmikroskop ist ein Lichtmikroskop, das polarisiertes Licht zur Abbildung verwendet. Es wird zur Untersuchung optisch anisotroper Objekte eingesetzt.

Zusätzlich zu einem normalen Lichtmikroskop enthält ein Polarisationsmikroskop zwei Polarisationsfilter und einen meist drehbaren Objekttisch. Polarisationsmikroskope arbeiten üblicherweise im Durchlichtmodus, obwohl es auch Auflicht-Polarisationsmikroskope gibt. Bei den Durchlicht-Polarisationsmikroskopen befindet sich unterhalb des Objekttisches ein Polarisationsfilter, auch Polarisator oder Primärfilter genannt, der das Licht der Lichtquelle des Mikroskops linear polarisiert, also nur Licht durchlässt, das in einer Schwingungsebene schwingt. Diese Schwingungsrichtung ist parallel zum Polarisator orientiert. Oberhalb des Objekttisches befindet sich ein zweiter Polarisationsfilter, der als Analysator oder Sekundärfilter bezeichnet wird und gegenüber dem ersten um 90° gedreht ist. Die Schwingungsrichtung des vorher linear polarisierten Lichtes ist dadurch genauso orientiert, dass es vom Analysator vollständig blockiert wird. Es besitzt keine Anteile, die in der Analysatorrichtung schwingen, daher erscheint das Bild schwarz. Die Anordnung von Primär- und Sekundärfilter wird "*gekreuzte Polarisatoren"* genannt.

Befindet sich auf dem Objekttisch zwischen den beiden Polarisationsfiltern eine Probe, so können sich die optischen Bedingungen ändern. Manche chemischen Verbindungen, zum Beispiel Minerale, haben unter bestimmten Bedingungen die Eigenschaft die Schwingungsebene des Lichts zu drehen, sie werden als doppelbrechend oder optisch anisotrop bezeichnet. Durch die Änderung der Polarisationsebene kommt es nicht mehr zur vollständigen Auslöschung - ein Teil des Lichtes dringt durch den Analysator und entsprechende Strukturen werden sichtbar. Auch ist es möglich, durch Interferenz auftretende Farben zu beobachten. Optisch isotrope Materialien bleiben hingegen dunkel.

Die Anwesenheit von Vesikeln, insbesondere von multilamellaren Vesikeln (MLV), kann durch Betrachtung einer Probe des Mittels (A) durch ein Polarisationsmikroskop, beispielsweise mit 400-facher Vergrößerung, nachgewiesen werden. Bevorzugt wird das Mittel (A) 24 Stunden nach seiner Herstellung unter dem Polarisationsmikroskop untersucht.

Da die Vesikel, insbesondere die multilamellaren Vesikel, optisch anisotrope Strukturen darstellen, lassen sich bei Anwesenheit der Vesikel speziell geformte, helle kreuzförmige Areale, die auch "Malteserkreuz-Muster" genannt werden, beobachten.

Als Polarisationsmikroskop kann beispielsweise ein Mikroskop von Zeiss (Axio Scope A2) mit verschiedenen Linsen und mit Polarisationsfilter eingesetzt werden.

Werden bei Untersuchung einer Probe unter dem Polarisationsmikroskope Malteserkreuze mit einer Größe von 1 bis zu 20 µm (Mikrometer) beobachtet, so lässt das spezielle Malteserkreuz-Muster auf die Anwesenheit von multilamellaren Vesikeln schließen.

### Durchmesser der unilamellaren und multilamellaren Vesikel

Unilamellare und multilamellare Vesikel unterscheiden sich insbesondere durch ihren Durchmesser.

Multilamellare Vesikel besitzen üblicherweise einen Durchmesser von 100 nm (Nanometer) bis zu 6 µm (Mikrometer), bevorzugt von 100 nm (Nanometer) bis zu 2 µm (Mikrometer). Unilamellare Vesikel hingegen haben einen kleineren Durchmesser von ca. 50 nm (Nanometer) bis typischerweise ca. 100 nm.

Im Zuge der zu dieser Erfindung führenden Arbeiten konnte beobachtet werden, dass durch das erfindungsgemäße Verfahren hergestellte Mittel (A), welche multilamellare Vesikel (MLV) enthielten, eine besonders hohe Salztoleranz und eine besonders gute Stabilität zeigten. Ohne an diese Theorie gebunden zu sein, wurde vermutet, dass die Ursache für diese verbesserten Eigenschaften in dem größeren Durchmesser der Vesikel liegt. Der größere Durchmesser der multilamellaren Vesikel (MLV) scheint für eine Erhöhung der Viskosität des Mittels verantwortlich zu sein, die wiederum die Stabilität des Mittels positiv beeinflusst.

Als ganz besonders bevorzugt hat es sich herausgestellt, wenn die multilamellaren Vesikel einen mittleren Durchmesser von 0,2 bis 6,0 µm (Mikrometer), bevorzugt von 0,2 bis 2,0 µm (Mikrometer) besitzen.

In einer besonders bevorzugten Ausführungsform ist ein erfindungsgemäßes Mittel (A) dadurch gekennzeichnet, dass es multilamellare Vesikel (MLV) mit einem mittleren Durchmesser von 0,02 bis 6,0 µm (Mikrometer), bevorzugt von 0,2 bis 2,0 µm (Mikrometer) umfasst.

Unter dem Vesikeldurchmesser im Sinne der vorliegenden Erfindung ist der mittlere Vesikeldurchmesser zu verstehen.

Der mittlere Durchmesser von Vesikeln kann erfindungsgemäß mittels dynamischer Lichtstreuung gemessen werden. Bei der dynamischen Lichtstreuung handelt es sich um eine Methode, bei der das Streulicht eines Laser an einer gelösten bzw. suspendierten Probe analysiert wird, um den hydrodynamischen Radius der Moleküle (bzw. im vorliegenden Fall der Vesikel) zu bestimmten. Die dynamische Lichtstreuung ist auch unter den Bezeichnungen Photonenkorrelationsspektroskopie oder quasielastische Lichtstreuung bekannt. Diese Methode ist insbesondere bei Vesikeldurchmessern von bis zu 2 µm (Mikrometern) geeignet.

Für die Messung der dynamischen Lichtstreuung kann beispielsweise ein Goniometer eingesetzt werden. Dabei befindet sich auf einem feststehenden Arm die Lasereinheit, und auf einem schwenkbaren Arm der Detektor, meist ein Sekundärelektronenvervielfacher (SEV) oder eine Avalanche-Photodiode (APD). In der Mitte der Anordnung befindet sich die Messzelle. Die Messung kann beispielsweise mit dem Gerät Zetasizer Nano ZS der Firma Malvern durchgeführt werden.

### C₈-C₃₀-Fettalkohole

Für die Ausbildung der Vesikel wesentlich ist die Anwesenheit mindestens eines C₈-C₃₀-Fettalkohols (a1). Deshalb enthalten die durch das erfindungsgemäße Verfahren hergestellten Mittel als wesentlichen Inhaltsstoff (a1) einen oder mehrere C₈-C₃₀-Fettalkohole. Bei den C₈-C₃₀-Fettalkoholen kann es sich um gesättigte, ein- oder mehrfach ungesättigte, lineare oder verzweigte Fettalkohole mit 8 bis 30 C-Atomen handeln.

Beispiele für bevorzugte lineare, gesättigte C₈-C₃₀-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9Z)-Octadec-9-en-1-ol (Oleylalkohol), (9E)-Octadec-9-en-1-ol (Elaidylalkohol), (9Z,12Z)-Octadeca-9,12-dien-1-ol (Linoleylalkohol), (9Z,12Z,15Z)-Octadeca-9,12,15-trien-1-ol (Linolenoylalkohol), Gadoleylalkohol ((9Z)-Eicos-9-en-1-ol), Arachidonalkohol ((5Z,8Z,11Z,14Z)-Eicosa-5,8,11,14-tetraen-1-ol), Erucylalkohol ((13Z)-Docos-13-en-1-ol) und/oder Brassidylalkohol ((13*E*)-Docosen-1-ol).

Die bevorzugten Vertreter für verzweigte Fettalkohole sind 2-Octyl-dodecanol, 2-Hexyl-Dodecanol und/oder 2-Butyl-dodecanol.

In einer besonders bevorzugten Ausführungsform enthält das durch das erfindungsgemäße Verfahren hergestellte Mittel mindestens einen linearen, gesättigten C₈-C₃₀-Fettalkohol. Besonders bevorzugt werden die linearen, gesättigten C₈-C₃₀-Fettalkohole in bestimmten Mengenbereichen im erfindungsgemäßen Mittel (A) eingesetzt.

Besonders stabile Mittel werden erhalten, wenn sie - bezogen auf ihre Gesamtgewicht - (a1) einen oder mehrere lineare, gesättigte C₈-C₃₀-Fettalkohole in einer Gesamtmenge von 5,0 bis 25,0 Gew.%, bevorzugt von 7,0 bis 20,0 Gew.-%, weiter bevorzugt von 10,0 bis 18,0 Gew.-% und besonders bevorzugt von 12,0 bis 16,0 Gew.-% enthalten.

In einer besonders bevorzugten Ausführungsform ist ein erfindungsgemäße Verfahren dadurch gekennzeichnet, dass das Mittel (A) - bezogen auf das Gesamtgewicht des Mittels (A) - (a1) einen oder mehrere lineare, gesättigte C₈-C₃₀-Fettalkohole in einer Gesamtmenge von 5,0 bis 25,0 Gew.%, bevorzugt von 7,0 bis 20,0 Gew.-%, weiter bevorzugt von 10,0 bis 18,0 Gew.-% und besonders bevorzugt von 12,0 bis 16,0 Gew.-% enthält.

Es hat sich weiterhin herausgestellt, dass die Stabilität und Salztoleranz der Formulierungen noch weiter verbessert werden kann, wenn die durch das erfindungsgemäße Verfahren hergestellten Mittel mindestens einen verzweigten C₈-C₃₀-Fettalkohol enthalten. Auch die verzweigten Fettalkohole werden bevorzugt in bestimmten Mengenbereichen im erfindungsgemäßen Mittel eingesetzt, die im Bereich von 1,0 bis 9,0 Gew.-%, bevorzugt von 1,3 bis 7,0 Gew.-%, weiter bevorzugt von 1,6 bis 5,0 Gew.-% und besonders bevorzugt von 1,9 bis 3,0 Gew.-% liegen. Hierbei beziehen sich alle Angaben in Gew.-% auf die Gesamtmenge der verzweigten C₈-C₃₀-Fettalkohole im Mittel eingesetzten C₈-C₃₀-Fettalkohole, die zum Gesamtgewicht des Mittels (A) in Relation gesetzt wird.

In einer besonders bevorzugten Ausführungsform ist ein erfindungsgemäße Verfahren dadurch gekennzeichnet, dass das Mittel (A) - bezogen auf das Gesamtgewicht des Mittels (A) - (a1) einen oder mehrere verzweigte, gesättigte C₈-C₃₀ Fettalkohole in einer Gesamtmenge von 1,0 bis 9,0 Gew.-%, bevorzugt von 1,3 bis 7,0 Gew.-%, weiter bevorzugt von 1,6 bis 5,0 Gew.-% und besonders bevorzugt von 1,9 bis 3,0 Gew.-% enthält.

In einer besonders bevorzugten Ausführungsform ist ein erfindungsgemäßes Verfahren Mittel (A) dadurch gekennzeichnet, dass das Mittel (A) - bezogen auf das Gesamtgewicht des Mittels (A) - (a1) 1,0 bis 9,0 Gew.-%, bevorzugt von 1,3 bis 7,0 Gew.-%, weiter bevorzugt von 1,6 bis 5,0 Gew.% und besonders bevorzugt von 1,9 bis 3,0 Gew.-% 2-Octyldodecanol enthält.

In einer Ausführungsform kann das durch das erfindungsgemäße Verfahren hergestellte Mittel (A) lineare, gesättigte C₈-C₃₀-Fettalkohole oder verzweigte gesättigte C₈-C₃₀ Fettalkohole enthalten. Im Hinblick auf die Stabilität des Mittels (A) hat es sich jedoch als ganz besonders vorteilhaft herausgestellt, wenn das Mittel (A) sowohl lineare, gesättigte C₈-C₃₀-Fettalkohole als auch verzweigte gesättigte C₈-C₃₀ Fettalkohole enthält.

So erwiesen sich beispielsweise die Färbemittel (A) als überaus lagerstabil, die nicht nur bis zu 6 Gew.-% an farbverändernden Salzen, sondern auch 13,0 Gew.-% Cetearylalkohol und 2,0 Gew.-% 2-Octyldodecanol enthielten.

### Ethoxylierte Fettalkohole der Formel (I)

Ebenfalls einen wesentlichen Beitrag für die Ausbildung stabiler uni- und multilamellarer Vesikel leisten die ethoxylierten Fettalkohole der Formel (I). Aus diesem Grund enthalten die über das erfindungsgemäße Verfahren hergestellten Mittel als weiteren wesentlichen Inhaltsstoff (a2) mindestens einen ethoxylierten Fettalkohol der Formel (I), 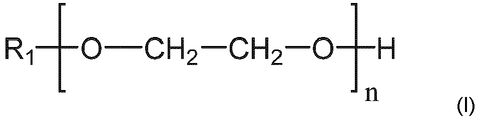 worin
R1 für eine lineare oder verzweigte, gesättigte oder ungesättigte C₈-C₃₀-Alkylgruppe steht und
n für eine ganze Zahl von 10 bis 100 steht.

Bei den Verbindungen der Formel (I) handelt es sich um nichtionische Verbindungen. Die Verbindungen der Formel (I) stellen ethoxylierte C₈-C₃₀-Fettalkohole mit einem Ethoxylierungsgrad von 10 bis 100 dar.

Je höher der Ethoxylierungsgrad ist, desto höher ist auch die Polarität der entsprechenden ethoxylierten Fettalkohole. Prinzipiell lassen sich sowohl niedrig ethoxylierte Fettalkohole (mit n bis zu 10) als auch sehr hoch ethoxylierte Fettalkohole (mit n bis zu hundert) in den erfindungsgemäßen Mitteln einsetzen.

Im Hinblick auf die Lösung der erfindungsgemäßen Aufgabenstellung als besonders gut geeignet erwiesen hat sich in diesem Zusammenhang der Einsatz von Verbindungen der Formel (I), in denen R1 für eine lineare oder verzweigte, gesättigte oder ungesättigte C₈-C₃₀-Alkylgruppe steht und n für eine ganze Zahl von 10 bis 30 steht.

In einer weiteren ganz besonders bevorzugten Ausführungsform ist ein erfindungsgemäßes Verfahren dadurch gekennzeichnet, dass das Mittel (A) (a2) mindestens einen ethoxylierten Fettalkohol der Formel (I) enthält, worin
R1 für eine lineare oder verzweigte, gesättigte oder ungesättigte C₈-C₃₀-Alkylgruppe steht und
n für eine ganze Zahl von 10 bis 30 steht.

Als entsprechende besonders geeignete ethoxylierte Fettalkohole mit einem Ethoxylierungsgrad von 10 bis 30 können beispielsweise Ceteareth-12 oder Ceteareth-20 genannt werden.

Auch die Verbindung(en) der Formel (I) werden bevorzugt in bestimmten Gesamtmengen im erfindungsgemäßen Mittel (A) eingesetzt. Besonders bevorzugt enthält das Mittel (A) einen oder mehrere ethoxylierte Fettalkohole der Formel (I) in einer Gesamtmenge von 0,5 bis 5,0 Gew.-%, bevorzugt von 1,0 bis 4,5 Gew.-%, weiter bevorzugt von 1,5 bis 4,0 Gew.-% und besonders bevorzugt von 2,0 bis 3,5 Gew.-%. Auch in diesem Zusammenhang sind alle Angaben in Gew.-% auf die Gesamtmenge aller im Mittel (A) enthaltenen ethoxylierten Fettalkohole der Formel (I) bezogen, die zum Gesamtgewicht des Mittels in Relation gesetzt wird.

In einer weiteren ganz besonders bevorzugten Ausführungsform ist ein erfindungsgemäßes Verfahren dadurch gekennzeichnet, dass das Mittel (A) - bezogen auf das Gesamtgewicht des Mittels (A) - (a2) einen oder mehrere ethoxylierte Fettalkohole der Formel (I) in einer Gesamtmenge von 0,5 bis 5,0 Gew.-%, bevorzugt von 1,0 bis 4,5 Gew.-%, weiter bevorzugt von 1,5 bis 4,0 Gew.% und besonders bevorzugt von 2,0 bis 3,5 Gew.-% enthält.

### Anionische Tenside der Formel (II)

Einen ganz besonderen Beitrag für die Ausbildung stabiler uni- und multilamellarer Vesikel leisten die anionischen Tenside der Formel (II). Aus diesem Grund enthalten die über das erfindungsgemäße Verfahren hergestellten Mittel als weiteren wesentlichen Inhaltsstoff (a3) mindestens ein anionisches Tensid der Formel (II), 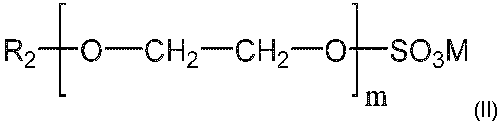 worin
R2 für eine lineare oder verzweigte, gesättigte oder ungesättigte C₈-C₃₀-Alkylgruppe steht,
m für eine ganze Zahl von 0 bis 10 steht und
M für ein Wasserstoffatom, ein Äquivalent eines Natriumions, eines Kaliumions oder eines Ammoniumions (NH₄⁺) steht.

Bei den anionischen Tensiden der Formel (II) kann es sich um Fettalkoholsulfate (im Fall von m = 0) handeln, wobei R2 den Fettalkoholrest (d.h. eine C₈-C₃₀-Alkylgruppe) des Tensids darstellt. Weiterhin kann es sich bei den anionischen Tensiden der Formel (II) auch um ethoxylierte Fettalkoholsulfate handeln, wobei die Zahl m den Ethoxylierungsgrad darstellt und für eine ganze Zahl von 1 bis 10 stehen kann.
Die Gruppierung M stellt das Gegenion zur Neutralisation des Sulfatrestes dar und kann für ein Wasserstoffatom stehen, in diesem Fall liegt die Verbindung der Formel (II) in Form ihrer Säure vor. Im wässrigen Trägermedium des Mittels steht die protonierte Form mit ihrem Anion im Gleichgewicht. Die Gruppierung M kann weiterhin auch für ein Natriumion (Na⁺), ein Kaliumion (K⁺) oder für ein Ammoniumion (NH₄⁺) stehen.

Zur Herstellung von besonders stabilen Formulierungen mit unilamellaren und multilamellaren Vesikeln hat es sich als ganz besonders vorteilhaft erwiesen, wenn die erfindungsgemäßen Mittel sowohl ein nicht ethoxyliertes Fettalkoholsulfat (d.h. eine Verbindung der Formel (II), bei der m für die Zahl 0 steht) als auch ein ethoxyliertes Fettalkoholsulfat (d.h. eine Verbindung der Formel (II), bei der m für die Zahl 1 bis 10, besonders bevorzugt für die Zahl 1 bis 5, steht.

In einer weiteren ganz besonders bevorzugten Ausführungsform ist ein erfindungsgemäßes Verfahren dadurch gekennzeichnet, dass das Mittel (A)
(a31) mindestens ein erstes anionisches Tensid der Formel (II) enthält, worin
   R2 für eine lineare, gesättigte C₈-C₃₀-Alkylgruppe steht und
   m für die Zahl 0 steht, und
(a32) mindestens ein zweites anionisches Tensid der Formel (II) enthält, worin
   R2 für eine lineare, gesättigte C₈-C₃₀-Alkylgruppe steht und
   m für eine ganze Zahl von 1 bis 5 steht.

Der Fachmann kennt die anionischen Tenside der Formel (II) unter dem allgemeinen Begriff Fettalkoholsulfate (z.B. Natriumlaurylsulfat, Natriumcetylsulfat, Natriumstearylsulfat, Natriumcetearylsulfat) oder Fettalkoholethersulfate (z.B. Natriumlaurethsulfat usw.). Diese anionischen Tenside fallen im Sinne der vorliegenden Erfindung explizit nicht unter die Gruppe der farbverändernde Sulfat-Salze, da das Sulfat im Fall der anionischen Tenside Teil des organischen Restes ist, unter farbverändernde Salzen (a5) jedoch erfindungsgemäß ausschließlich kationische Verbindungen mit Sulfat-Anion (d.h. SO₄²⁻) verstanden werden. Zudem entfalten die anionischen Tenside (a3) auf Keratinfasern keinerlei farbverändernde Eigenschaften.

Auch die Verbindung(en) der Formel (II) werden bevorzugt in bestimmten Gesamtmengen im erfindungsgemäßen Mittel (A) eingesetzt. Besonders bevorzugt enthält das Mittel (A) ein oder mehrere anionische Tenside der Formel (II) in einer Gesamtmenge von 0,5 bis 4,8 Gew.-% bevorzugt von 0,5 bis 4,2 Gew.-%, weiter bevorzugt von 0,5 bis 3,8 Gew.-% und besonders bevorzugt von 1,0 bis 3,0 Gew.-%. Auch in diesem Zusammenhang sind alle Angaben in Gew.-% auf die Gesamtmenge aller im Mittel (A) enthaltenen anionischen Tenside der Formel (II) bezogen, die zum Gesamtgewicht des Mittels in Relation gesetzt wird.

In einer weiteren ganz besonders bevorzugten Ausführungsform ist ein erfindungsgemäßes Verfahren dadurch gekennzeichnet, dass das Mittel (A) - bezogen auf das Gesamtgewicht des Mittels (A) - (a3) ein oder mehrere anionische Tenside der Formel (II) in einer Gesamtmenge von 0,5 bis 4,8 Gew.-% bevorzugt von 0,5 bis 4,2 Gew.-%, weiter bevorzugt von 0,5 bis 3,8 Gew.-% und besonders bevorzugt von 1,0 bis 3,0 Gew.-% enthält.

### Farbverändernde Salze aus der Gruppe der Sulfate, der Hydrogensulfate, der Chloride und/oder der Bromide (a4)

Bei den über das erfindungsgemäße Verfahren hergestellten Mitteln handelt es sich um Mittel zur Farbveränderung von keratinischen Fasern, daher enthalten die Mittel mindestens eine farbverändernde Verbindung. In die Gruppe der farbverändernden Verbindungen fallen erfindungsgemäß direktziehende Farbstoffe und insbesondere Oxidationsfarbstoffvorprodukte.

Oxidationsfarbstoffvorprodukte können in Entwickler und Kuppler unterteilt werden, wobei die Entwickler aufgrund ihrer größeren Empfindlichkeit gegenüber Sauerstoff meist in Form ihrer physiologisch verträglichen Farbverändernde Salze (z.B. in Form ihrer Sulfate, Hydrogensulfate, Chloride oder Bromide) eingesetzt werden.

Kupplerkomponenten bilden im Rahmen der oxidativen Färbung allein keine signifikante Färbung aus, sondern benötigen stets die Gegenwart von Entwicklerkomponenten. Da Kuppler gegenüber Sauerstoff nicht so empfindlich sind wie Entwickler, können sie zwar ebenfalls in Form ihrer Farbverändernde Salze in den Zubereitungen eingesetzt werden, werden oftmals aber auch in freier Form (d.h. nicht in Salzform) eingesetzt.

Im Zuge der zu dieser Erfindung führenden Arbeiten hat sich herausgestellt, dass insbesondere Nuancen mit hohem Mengenanteil an Oxidationsfarbstoffvorprdodukten, d.h. Braun-Nuancen, Schwarz-Nuancen oder andere dunkle Nuancen, sehr gut durch die erifndungsgemäßen Mittel stabilisiert werden können. Dieser Effekt ist besonders dann ausgeprägt, wenn Oxidationsfarbstoffvorprodukte in Form ihrer physiologisch verträglichen Salze eingesetzt werden.

Bevorzugte physiologisch verträgliche farbverändernde Salze von Entwicklern sind beispielsweise Phenylendiamin Sulfat, Phenylendiamin Monohydrochlorid, Phenylendiamin Dihydrochlorid, p-Toluylendiamin Sulfat, p-Toluylendiamin Monohydrochlorid, p-Toluylendiamin Dihydrochlorid, 2-(2-Hydroxyethyl)-p-phenylendiamin Sulfat, 2-(2-Hydroxyethyl)-p-phenylendiamin Monohydrochlorid, 2-(2-Hydroxyethyl)-p-phenylendiamin Dihydrochlorid, N,N-Bis-(2-hydroxyethyl)-p-phenylendiamin Sulfat, N,N-Bis-(2-hydroxyethyl)-p-phenylendiamin Monohydrochlorid, N,N-Bis-(2-hydroxyethyl)-p-phenylendiamin Dihydrochlorid, 2-Methoxymethyl-p-phenylendiamin Sulfat, 2-Methoxymethyl-p-phenylendiamin Monohydrochlorid, 2-Methoxymethyl-p-phenylendiamin Dihydrochlorid, p-Aminophenol Hydrogensulfat, p-Aminophenol Monohydrochlorid, 4-Amino-3-methylphenol Hydrogensulfat, 4-Amino-3-methylphenol Chlorid, 2,4,5,6-Tetraaminopyrimidin Monosulfat, 2,4,5,6-Tetraaminopyrimidin Disulfat, 2,4,5,6-Tetraaminopyrimidin Monohydrochlorid, 2,4,5,6-Tetraaminopyrimidin Dihydrochlorid, 2,4,5,6-Tetraaminopyrimidin Trihydrochlorid, 2,4,5,6-Tetraaminopyrimidin Tetrahydrochlorid, 4-Hydroxy-2,5,6-triaminopyrimidin Sulfat, 4-Hydroxy-2,5,6-triaminopyrimidin Monohydrochlorid, 4-Hydroxy-2,5,6-triaminopyrimidin Diydrochlorid, 4-Hydroxy-2,5,6-triaminopyrimidin Trihydrochlorid, 4,5-Diamino-1-(2-hydroxyethyl)-1H-pyrazol Sulfat, 4,5-Diamino-1-(2-hydroxyethyl)-1H-pyrazol Monohydrochlorid und/oder 4,5-Diamino-1-(2-hydroxyethyl)-1H-pyrazol Dihydrochlorid.

In einer weiteren ganz besonders bevorzugten Ausführungsform ist ein erfindungsgemäßes Verfahren dadurch gekennzeichnet, dass das Mittel (A) (a4) ein oder mehrere farbverändernde Salze aus der Gruppe aus Phenylendiamin Sulfat, Phenylendiamin Monohydrochlorid, Phenylendiamin Dihydrochlorid, p-Toluylendiamin Sulfat, p-Toluylendiamin Monohydrochlorid, p-Toluylendiamin Dihydrochlorid, 2-(2-Hydroxyethyl)-p-phenylendiamin Sulfat, 2-(2-Hydroxyethyl)-p-phenylendiamin Monohydrochlorid, 2-(2-Hydroxyethyl)-p-phenylendiamin Dihydrochlorid, N,N-Bis-(2-hydroxyethyl)-p-phenylendiamin Sulfat, N,N-Bis-(2-hydroxyethyl)-p-phenylendiamin Monohydrochlorid, N,N-Bis-(2-hydroxyethyl)-p-phenylendiamin Dihydrochlorid, 2-Methoxymethyl-p-phenylendiamin Sulfat, 2-Methoxymethyl-p-phenylendiamin Monohydrochlorid, 2-Methoxymethyl-p-phenylendiamin Dihydrochlorid, p-Aminophenol Hydrogensulfat, p-Aminophenol Monohydrochlorid, 4-Amino-3-methylphenol Hydrogensulfat, 4-Amino-3-methylphenol Chlorid, 2,4,5,6-Tetraaminopyrimidin Monosulfat, 2,4,5,6-Tetraaminopyrimidin Disulfat, 2,4,5,6-Tetraaminopyrimidin Monohydrochlorid, 2,4,5,6-Tetraaminopyrimidin Dihydrochlorid, 2,4,5,6-Tetraaminopyrimidin Trihydrochlorid, 2,4,5,6-Tetraaminopyrimidin Tetrahydrochlorid, 4-Hydroxy-2,5,6-triaminopyrimidin Sulfat, 4-Hydroxy-2,5,6-triaminopyrimidin Monohydrochlorid, 4-Hydroxy-2,5,6-triaminopyrimidin Diydrochlorid, 4-Hydroxy-2,5,6-triaminopyrimidin Trihydrochlorid, 4,5-Diamino-1-(2-hydroxyethyl)-1H-pyrazol Sulfat, 4,5-Diamino-1-(2-hydroxyethyl)-1H-pyrazol Monohydrochlorid und/oder 4,5-Diamino-1-(2-hydroxyethyl)-1H-pyrazol Dihydrochlorid enthält.

Bei Oxidationsfarbstoffvorprodukten vom Entwicklertyp handelt es sich üblicherweise um Derviate des p-Phenylendiamins, das p-Aminophenols oder um heterozyklische Verbindungen mit mindestens einer, bevorzugt mindestens zwei Aminogruppen. Zur Überführung in ihre Salze werden die in diesen Strukturen enthaltenen Aminogruppen protoniert und besitzen zur Neutralisation dieser positiven Ladung die entsprechenden Äquivalente an Sulfat-Anionen, Hydrogensulfat-Anionen, Chlorid-Anionen und/oder Bromid-Anionen.

Bei p-Toluylendiamin Sulfat handelt es sich beipsielsweise um die Verbindung Toluylendiamin x H₂SO₄. Beide Aminogruppen liegen protoniert (in Form von Ammoniumionen) vor und die nun im Molekül enthaltenen beiden kationischen Ladungen werden durch ein Sulfat-Anion (SO₄²⁻) neutralisiert. Bei p-Toluylendiamin Monohydrochlorid handelt es sich demnach um die Verbindung Toluylendiamin x HCl. Eine der beiden Aminogruppen liegt protoniert vor und besitzt ein Chlorid als Gegenion. Bei p-Toluylendiamin Dihydrochlorid handelt es sich demnach um die Verbindung Toluylendiamin x 2 HCl. Beide Aminogruppen liegt protoniert vor und besitzten zwei Chloride als Gegenion. Die Salze der weiteren Oxidationsfarbstoffe vom Entwicklertyp setzen sich in analoger Weise zusammen.

Abhängig vom gewünschten Farbresultat werden Oxidationsfarbstoffvorprodukte vom Entwickler- und vom Kupplertyp in unterschiedlich hohen Mengenanteilen im Färbemittel eingesetzt.

Wird die Färbung in einer Blondnuance gewünscht, so genügt meist der Einsatz Oxidationsfarstoffvorprodukten in einer Gesamtmenge unterhalb von 0,3 Gew.-%.

Wünscht der Anwender jedoch die Färbung in einem sehr dunklen Farbton, beispielsweise in einer Dunkelbraun-Nuance oder in einer Schwarz-Nuance, so macht dies den Einsatz von Oxidationsfarbsotffvorprodukten in einer Gesamtmenge von mindestens 2,0 Gew.-%, oft 3,0 Gew.%, und bei besonders dunklen Nuancen (schwarz) sogar oberhalb von 4,5 Gew.-% (bezogen auf das Gesamtgewicht des Färbemittels (A) erforderlich.

Je höher der Farbstoffgehalt ist, desto schwieriger ist es, das Mittel zu stabilisieren. In diesem Zusammenhang hat sich herausgestellt, dass vor allem die Stabilisierung von Braun- bzw. Schwarz-Nuancen durch die Ausbildung von uni- und multilamellaren Vesikeln sehr gut möglich ist. Die durch das erfindungsgemäße Verfahren hergestellten Mittel (A) enthalten daher ein oder mehrere farbverändernde farbverändernde Salze (a4) aus der Gruppe der Sulfate, der Hydrogensulfate, der Chloride und/oder der Bromide in einer Gesamtmenge von 0,9 bis 3,4 Gew.-% und ganz besonders bevorzugt von 2,1 bis 3,4 Gew.-%. Auch in diesem Zusammenhang sind alle Angaben in Gew.-% auf die Gesamtmenge aller im Mittel farbverändernden Salze (a4) bezogen, die zum Gesamtgewicht des Mittels in Relation gesetzt wird.

In einer weiteren ganz besonders bevorzugten Ausführungsform ist ein erfindungsgemäßes Verfahren dadurch gekennzeichnet, dass das Mittel (A) - bezogen auf das Gesamtgewicht des Mittels (A) - (a4) ein oder mehrere farbverändernde farbverändernde Salze aus der Gruppe der Sulfate, der Hydrogensulfate, der Chloride und/oder der Bromide in einer Gesamtmenge von 2,1 bis 3,4 Gew.-% enthält.

Im Rahmen einer weiteren Ausführungsform als ganz besonders bevorzugt erwiesen hat sich daher ein Verfahren zur Herstellung eines Mittels (A) zur Farbveränderung von keratinischen Fasern, insbesondere menschlichen Haaren, wobei das Mittel (A) in einem wässrigen Medium unilamellare Vesikel (ULV) und/oder multilamellare Vesikel (MLV) umfasst, wobei das Mittel - bezogen auf sein Gesamtgewicht - enthält
(a11) einen oder mehrere lineare, gesättigte C₈-C₃₀-Fettalkohole in einer Gesamtmenge von 12,0 bis 16,0 Gew.-%, und
(a12) einen oder mehrere verzweigte, gesättigte C₈-C₃₀ Fettalkohole in einer Gesamtmenge von 1,9 bis 3,0 Gew.-% und
(a2) einen oder mehrere ethoxylierte Fettalkohole der Formel (I) in einer Gesamtmenge von 2,0 bis 3,5 Gew.-%, 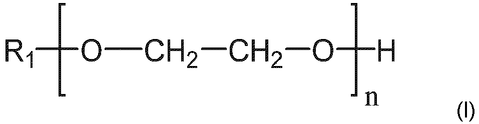 worin
   R1 für eine lineare oder verzweigte, gesättigte oder ungesättigte C₈-C₃₀-Alkylgruppe steht und
   n für eine ganze Zahl von 10 bis 100 steht
(a3) ein oder mehrere anionische Tenside der Formel (II) in einer Gesamtmenge von 1,0 bis 3,0 Gew.-%, 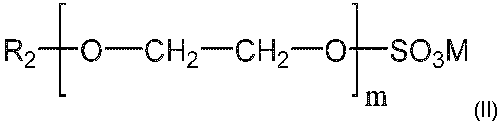 worin
   R2 für eine lineare oder verzweigte, gesättigte oder ungesättigte C₈-C₃₀-Alkylgruppe steht,
   m für eine ganze Zahl von 0 bis 10 steht und
   M für ein Wasserstoffatom, ein Äquivalent eines Natriumions, eines Kaliumions oder eines Ammoniumions (NH₄⁺) steht,
(a4) ein oder mehrere farbverändernde Salze aus der Gruppe der Sulfate, der Hydrogensulfate, der Chloride und/oder der Bromide in einer Gesamtmenge von 2,1 bis 3,4 Gew.-%.

Im Rahmen einer weiteren Ausführungsform als ganz besonders bevorzugt erwiesen hat sich daher ein Verfahren zur Herstellung eines Mittels (A) zur Farbveränderung von keratinischen Fasern, insbesondere menschlichen Haaren, wobei das Mittel (A) in einem wässrigen Medium multilamellare Vesikel (MLV) umfasst, wobei das Mittel - bezogen auf sein Gesamtgewicht - enthält
(a11) einen oder mehrere lineare, gesättigte C₈-C₃₀-Fettalkohole in einer Gesamtmenge von 12,0 bis 16,0 Gew.-%, und
(a12) einen oder mehrere verzweigte, gesättigte C₈-C₃₀ Fettalkohole in einer Gesamtmenge von 1,9 bis 3,0 Gew.-% und
(a2) einen oder mehrere ethoxylierte Fettalkohole der Formel (I) in einer Gesamtmenge von 2,0 bis 3,5 Gew.-%, 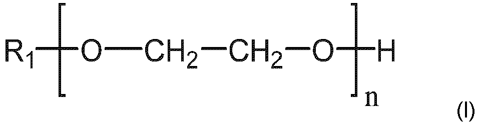 worin
   R1 für eine lineare oder verzweigte, gesättigte oder ungesättigte C₈-C₃₀-Alkylgruppe steht und
   n für eine ganze Zahl von 10 bis 100 steht
(a3) ein oder mehrere anionische Tenside der Formel (II) in einer Gesamtmenge von 1,0 bis 3,0 Gew.-%, 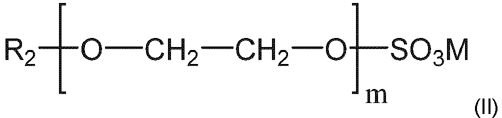 worin
   R2 für eine lineare oder verzweigte, gesättigte oder ungesättigte C₈-C₃₀-Alkylgruppe steht,
   m für eine ganze Zahl von 0 bis 10 steht und
   M für ein Wasserstoffatom, ein Äquivalent eines Natriumions, eines Kaliumions oder eines Ammoniumions (NH₄⁺) steht,
(a4) ein oder mehrere farbverändernde Salze aus der Gruppe der Sulfate, der Hydrogensulfate, der Chloride und/oder der Bromide in einer Gesamtmenge von 2,1 bis 3,4 Gew.-%.

Oxidationsfarbstoffvorprodukte vom Entwicklertyp können als alleinige farbverändernde Verbindungen im erfindungsgemäßen Mittel enthalten sein. Es ist erfindungsgemäß jedoch bevorzugt, wenn das Färbemittel (A) zusätzlich mindestens ein Oxidationsfarbstoffvorprodukt vom Kupplertyp (kurz Kuppler genannt) enthält.

Kupplerkomponenten bilden im Rahmen der oxidativen Färbung allein keine signifikante Färbung aus, sondern benötigen stets die Gegenwart von Entwicklerkomponenten. Kupplerkomponenten im Sinne der Erfindung erlauben mindestens eine Substitution eines chemischen Restes des Kupplers durch die oxidierte Form der Entwicklerkomponente. Dabei bilden sich kovalente Bindungen zwischen Kuppler- und Entwicklerkomponente aus.

Als erfindungsgemäß geeignete Kupplerkomponente wird bevorzugt mindestens eine Verbindung aus einer der folgenden Klassen ausgewählt:
- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin.

Gemische aus zwei oder mehreren Verbindungen aus einer oder mehreren dieser Klassen sind im Rahmen dieser Ausführungsform ebenso erfindungsgemäß.

In einer weiteren Ausführungsform ist ein erfindungsgemäßes Verfahren dadurch gekennzeichnet, dass das Mittel (A) mindestens ein Oxidationsfarbstoffvorprodukt vom Kuppler-Typ enthält, welches ausgewählt wird aus der Gruppe aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)-ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin und deren physiologisch verträglichen Farbverändernde Salzen.

Zusätzlich zu den Oxidationsfarbstoffvorprodukten oder anstatt dieser können die durch das erfindungsgemäße Verfahren hergestellten Färbemittel mindestens einen direktziehenden Farbstoff (D) enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Direktziehende Farbstoffe können in anionische, kationische und nichtionische direktziehende Farbstoffe unterteilt werden.

Kationische Farbstoffe, die in Form ihrer Sulfate, Hydrogensulfate, Chloride und/oder Bromide vorliegen, fallen im Sinne der vorliegenden Erfindung ebenfalls unter die Definition der farbgebenden Salze (a4).

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Farbverändernde Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe tragen mindestens eine negative Ladung und werden in der Literatur auch als Säurefarbstoffe bezeichnet. Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Kationische Farbstoffe zeichnen sich durch das Vorhandensein von mindestens einer positiven Ladung aus. In der englischen Literatur werden kationische Farbstoffe auch "basic dyes" genannt. Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Yellow 87, Basic Orange 31 und Basic Red 51.

Wie bereits zuvor beschrieben wird die Stabilisierung von Emulsionen oft umso schwieriger, je höher der Salzgehalt einer Emulsion ist. Daher lassen sich Emulsionen, in denen hohe Mengen an kationischen direktziehenden Farbstoffen eingesetzt werden, üblicherweise auch schwieriger stabilisieren als Emulsionen mit niedrigem Farbstoffgehalt oder Emulsionen, die nur nichtionische direktziehende Farbstoffe enthalten.

Im Zuge der durchgeführten Arbeiten hat sich gezeigt, dass sich die in Emulsionsform vorliegenden Färbemittel (A) insbesondere auch dann sehr gut stabilisieren ließen, wenn das Färbemittel (A) als farbgebende Salze (a4) direktziehende kationische Farbstoffe enthält, die in Form ihrer Sulfate, Hydrogensulfate, Chloride und/oder Bromide vorliegen.

Prinzipiell können die direktziehenden Farbstoffe in einer Gesamtmenge von 0,001 bis 10 Gew.-% im erfindungsgemäßen Färbemittel (A) enthalten sein.

Werden als direktziehende Farbstoffe jedoch kationische Farbstoffe in Form ihrer Sulfat-Salze, Hydrogensulfat-Salze, Chlorid-Salze und/oder Bromid-Salze eingesetzt, so fallen sie in die Gruppe der farbgebenden Salze (a4) und werden in den zuvor beschriebenen erfindungsgemäßen, bevorzugten und besonders bevorzugten Mindest- und Höchstmengen eingesetzt.

### Phosphorhaltige Verbindungen

Aus dem Stand der Technik ist bekannt, dass unilamellare bzw. multilamellare Vesikel sich leicht bei Einsatz von typischen Vesikelbildnern ausbilden. Als typische Vesikelbildner werden vor allem die aus biologischen Prozessen bekannten Phospholipide oder Phosphoaminolipide genannt. Diese phosphorhaltigen Vesikelbildner sind in der Regel teuer, so dass ständig nach effektiveren und ökonomischeren Methoden gesucht wird, auch ohne Einsatz dieser phosphorhaltigen Verbindungen uni- und multilamellare Vesikel ausbilden zu können.

Überraschenderweise hat sich herausgestellt, dass durch Einsatz der zuvor beschriebenen wesentlichen Inhaltsstoffe (a1), (a2), (a3) und (a4) möglich wird, auch ohne Verwendung der typischen phosphorhaltigen Verbindungen uni- und multilamellare Vesikel im erfindungsgemäßen Mittel (A) auszubilden.

Aus diesem Grund ist es ganz besonders bevorzugt, wenn das Mittel (A) - bezogen auf das Gesamtgewicht des Mittels (A) - phosphorhaltige Verbindungen in einer Gesamtmenge von weniger als 0,1 Gew.-%, bevorzugt von weniger als 0,05 Gew.-% enthält.

In einer weiteren ganz besonders bevorzugten Ausführungsform ist ein erfindungsgemäßes Verfahren dadurch gekennzeichnet, dass das Mittel (A) - bezogen auf das Gesamtgewicht des Mittels (A) - phosphorhaltige Verbindungen in einer Gesamtmenge von weniger als 0,1 Gew.-%, bevorzugt von weniger als 0,05 Gew.-% enthält.

Unter phosphorhaltigen Verbindungen werden im Sinne der vorliegenden Erfindung alle anorganischen und organischen Verbindungen verstanden, die ungeladen oder geladen sein können deren Strukturen als Strukturbestandteil mindestens ein Phosphoratom (entweder im organischen Teil und/oder im anorganischen Teil) umfassen.

### C₈-C₃₀-Fettsäuremonoglyceride, C₈-C₃₀-Fettsäurediglyceride C₈-C₃₀-Fettsäuretriglyceride (a5)

Für die Ausbildung der unilamellaren und multilamellaren Vesikel als besonders vorteilhaft hat es sich weiterhin herausgestellt, wenn die durch das erfindungsgemäße Verfahren hergestellten Mittel weiterhin zusätzlich mindestens ein C₈-C₃₀-Fettsäuremonoglycerid, ein C₈-C₃₀-Fettsäurediglycerid und/oder ein C₈-C₃₀-Fettsäuretriglycerid als weiteren besonders bevorzugten Inhaltsstoff (a5) enthalten.

In einer weiteren ganz besonders bevorzugten Ausführungsform ist ein erfindungsgemäßes Verfahren dadurch gekennzeichnet, dass das Mittel (A) (a5) mindestens ein C₈-C₃₀-Fettsäuremonoglycerid, ein C₈-C₃₀-Fettsäurediglycerid und/oder ein C₈-C₃₀-Fettsäuretriglycerid enthält.

Unter einem C₈-C₃₀-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte C₈-C₃₀-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem C₈-C₃₀-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die C₈-C₃₀-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)-Tetracos-15-ensäure].

Unter einem C₈-C₃₀-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Innerhalb der Gruppe der ein C₈-C₃₀-Fettsäuremonoglyceride, ein C₈-C₃₀-Fettsäurediglyceride und/oder ein C₈-C₃₀-Fettsäuretriglyceride haben die C₈-C₃₀-Fettsäuremonoglyceride die besten Eigenschaften gezeigt. Daher ist es ganz besonders bevorzugt im erfindungsgemäßen Mittel (a) mindestens ein C₈-C₃₀-Fettäuremonoglycerid einzusetzen.

Auch die Verbindung(en) aus der Gruppe (a5) werden bevorzugt in bestimmten Gesamtmengen -in dem durch das erfindungsgemäße Verfahren hergestellten Mittel (A) eingesetzt. Besonders bevorzugt enthält das Mittel (A) ein oder mehrere C₈-C₃₀-Fettsäuremonoglyceride, ein C₈-C₃₀-Fettsäurediglyceride und/oder ein C₈-C₃₀-Fettsäuretriglyceride in einer Gesamtmenge von 1,0 bis 6,0 Gew.-%, bevorzugt von 1,5 bis 5,5 Gew.-%, weiter bevorzugt von 2,0 bis 5,0 Gew.-% und ganz besonders bevorzugt von 2,5 bis 4,5 Gew.-% Auch in diesem Zusammenhang sind alle Angaben in Gew.-% auf die Gesamtmenge aller im Mittel (A) enthaltenen Verbindungen aus der Gruppe (a5) bezogen, die zum Gesamtgewicht des Mittels in Relation gesetzt wird.

Ganz besonders bevorzugt enthält das Mittel (A) ein oder mehrere C₈-C₃₀-Fettsäuremonoglyceride in einer Gesamtmenge von 1,0 bis 6,0 Gew.-%, bevorzugt von 1,5 bis 5,5 Gew.-%, weiter bevorzugt von 2,0 bis 5,0 Gew.-% und ganz besonders bevorzugt von 2,5 bis 4,5 Gew.-%.

In einer weiteren ganz besonders bevorzugten Ausführungsform ist ein erfindungsgemäßes Verfahren dadurch gekennzeichnet, dass das Mittel (A) - bezogen auf das Gesamtgewicht des Mittels (A) - (a5) ein oder mehrere C₈-C₃₀-Fettsäuremonoglyceride in einer Gesamtmenge von 1,0 bis 6,0 Gew.-%, bevorzugt von 1,5 bis 5,5 Gew.-%, weiter bevorzugt von 2,0 bis 5,0 Gew.-% und ganz besonders bevorzugt von 2,5 bis 4,5 Gew.-% enthält.

### Wasserphase

Die durch das erfindungsgemäße Verfahren hergestellten Mittel umfassen ein wässriges Medium, das ebenfalls zur Ausbildung der Vesikel beiträgt bzw. den hydrophilen Innen- und/oder Außenraum bildet, in dem sich die uni- und multilamellaren Vesikel befinden. Bevorzugt enthält das erfindungsgemäße Mittel (A) - bezogen auf sein Gesamtgewicht - Wasser in einer Menge von 60,0 bis 85,0 Gew.-%, bevorzugt von 65,0 bis 75,0 Gew.-%, weiter bevorzugt von 60,0 bis 80,0 Gew.-% und besonders bevorzugt von 65 bis 75,0 Gew.-%.

In einer weiteren ganz besonders bevorzugten Ausführungsform ist ein erfindungsgemäßes Verfahren dadurch gekennzeichnet, dass das Mittel (A) - bezogen auf das Gesamtgewicht das Mittels (A) - Wasser in einer Menge von 60,0 bis 85,0 Gew.-%, bevorzugt von 65,0 bis 75,0 Gew.%, weiter bevorzugt von 60,0 bis 80,0 Gew.-% und besonders bevorzugt von 65 bis 75,0 Gew.-% enthält.

### Weitere Inhaltsstoffe

Die durch das erfindungsgemäße Verfahren hergestellten Mittel (A) zusätzlich weitere Wirk-, Hilfs- und Zusatzstoffe enthalten, wie beispielsweise kationische Tenside, amphotere Tenside, anionische, nichtionische und/oder kationische Polymere, Strukturanten wie Glucose, Maleinsäure und Milchsäure, Alalisierungsmittel wie Ammoniak, Monoethanolamin, Kaliumhydroxid und Natriumhydroxid; Parfümöle, faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Farbverändernde Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Vitamine, Provitamine und Vitaminvorstufen; Pflanzenextrakte; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht des Mittels (A) eingesetzt.

### Verfahren zur Herstellung des Mittels (A)

Die erfindungsgemäßen Mittel (A) werden nach einem speziellen Verfahren hergestellt, durch das die Entstehung von uni- und multilamellaren Vesikeln gewährleistet werden kann.

Das Verfahren zur Herstellung eines Mittels (A), umfassent die folgenden Schritte
(I) Erhitzen eines Gemisches aus (a1) C₈-C₃₀-Fettalkohol, (a2) ethoxyliertem Fettalkohol der Formel (I), (a3) anionischem Tensid der Formel (II) und einer ersten Teilmenge Wasser auf eine Temperatur von 65 bis 85 °C in einem ersten Behältnis,
(II) Herstellung eines Gemisches aus einer zweiten Teilmenge Wasser und den farbverändernden Salzen aus der Gruppe der Sulfate, der Hydrogensulfate, der Chloride (a4) in einem zweiten Behältnis,
(III) Zugabe der in Schritt (II) erhaltenen Zubereitung zu der in Schritt (I) erhaltenen, auf 40 bis 60 °C temperierten Zubereitung unter konstantem Rühren mit einem Propeller-Rührwerk bei mindestens 500 Umdrehungen pro Minute,
(IV) Abkühlen des in Schritt (III) erhaltenen Gemisches unter ständigem Rühren auf 20 bis 40 °C.

Als besonders bevorzugt zur Herstellung unilamellarer und multilamellarer Vesikel hat sich der Eintrag von hohen Scherkräften in Schritt (III) des Verfahrens erwiesen. Entsprechend hohe Scherkräfte können beispielsweise durch Rühren der Formulierung mit einem Propellerrührwerk, beispielsweise mit mindestens 500 Umdrehungen pro Minute, weiter bevorzugt mit mindestens 800 Umdrehungen pro Minute, erzeugt werden.

Als besonders bevorzugt hat sich herausgestellt, wenn die
(III) Zugabe der in Schritt (II) erhaltenen Zubereitung zu der in Schritt (I) erhaltenen, auf 40 bis 60 °C temperierten Zubereitung unter konstantem Rühren mit einem Propeller-Rührwerk bei mindestens 800 Umdrehungen pro Minute erfolgt.

### Beispiele

Es wurden die folgenden Zusammensetzungen hergestellt (alle Angaben in Gew.-%)

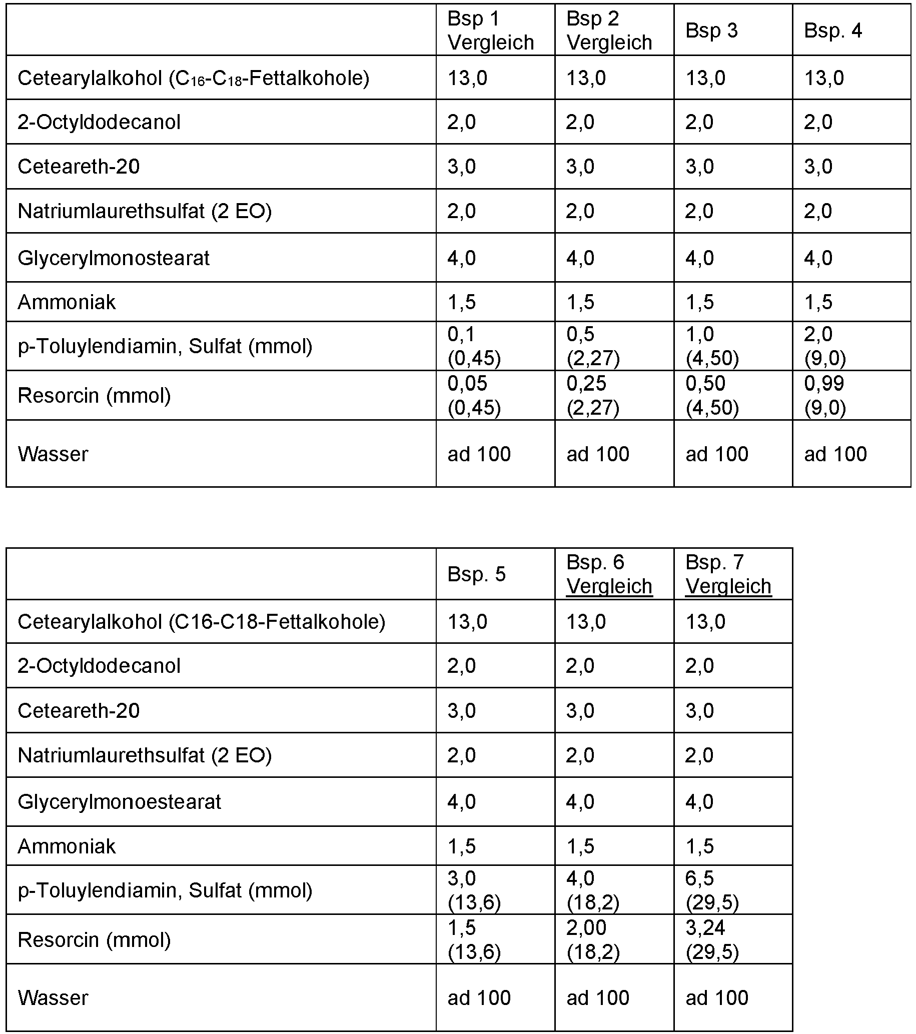

Die Formulierungen wurden wie folgt hergestellt:
Cetarylalkohol und 2-Octyldodecanol wurden zusammen mit Ceteareth-20, Natriumlaurethsulfat (2EO), Glycerylmonostearat und mit einer ersten Teilmenge des Wassers unter Rühren auf 75 °C erhitzt (Fettphase)

Dann wurden Ammoniak und die Oxidationsfarbstoffvorprodukte mit einer zweiten Teilmenge des Wassers bei 20 - 30 °C vermischt (Farbstoffphase).
Nachdem die Fettphase auf 50 °C abgekühlt war, wurde die auf 30 °C temperierte Farbstoffphase unter Rühren mit einem Propellerrührwerk bei 1000 Umdrehungen pro Minute in die Fettphase eingearbeitet.

Die auf diese Weise hergestellten Formulierungen wurden unter Rühren mit einem Propellerrührwerk bei 1000 Umdrehungen pro Minute auf Raumtemperatur abkühlen gelassen.

Nach dem Herstellen wurden alle Formulierungen im Hinblick auf ihre Stabilität bewertet

| | Anteil-Salze (Gew.-%) | 24 h nach Herstellung | 4 Wochen nach Herstellung | Stabilität |
|---|---|---|---|---|
| | | | | 1 = sehr gut |
| | | | | 6 = sehr schlecht |
| Bsp. 1 Vergleich | 0,1 | halb durchsichtiges Gel | gelig, viskos | 2 |
| Bsp. 2 Vergleich | 0,5 | halb durchsichtiges Gel | gelig, viskos | 2 |
| Bsp. 3 | 1,0 | weiße Creme | dicke Creme | 1 |
| Bsp. 4 | 2,0 | weiße Creme | dicke Creme | 1 |
| Bsp. 5 | 3,0 | weiße Creme | dicke Creme | 1 |
| Bsp. 6 Vergleich | 4,0 | weiße Creme | dicke Creme | 2 |
| Bsp. 7 Vergleich | 6,5 | weiße Creme | Separation von Tropfen, Phasentrennung, dünn | 5 |

Nach 24 Stunden wurden die Formulierungen unter dem Polarisationsmikroskop (Zeiss Axio Scope a2) untersucht:

| | Salze | |
|---|---|---|
| Bsp. 1 | 0,1 | lamellare Phase, hauptsächlich mit linearen, parallele Strukturen |
| Bsp. 3 | 1,0 | Malteserkreuze, ca. 4 - 5 µm (Mikrometer), multilamellare Vesikel |
| Bsp. 5 | 3,0 | Malteserkreuze, ca. 5 - 6 µm (Mikrometer), multilamellare Vesikel |
| Bsp. 6 | 4,0 | Malteserkreuze, ca. 7 - 20 µm (Mikrometer), multilamellare Vesikel |
| Bsp. 7 | 6,5 | Malteserkreuze, ca. 20 µm (Mikrometer) |

### Weitere Formulierunqsbeispiele

Es wurden die folgenden Farbcremes hergestellt (alle Angaben in Gew.-%).

| | FC1 Vergleich | FC2 Vergleich | FC3 |
|---|---|---|---|
| Carbomer (Polyacyrylsäure, Ammoniumsalz, Homo-Poylmer) | 0,2 | 0,2 | 0,2 |
| C16-C18-Fettalkoholsulfat, Natriumsalz | 0,7 | 0,7 | 0,7 |
| Natriumlaurethsulfat (C12-C14-Fettalkohol, 2-3 EO) | 1,3 | 1,3 | 1,3 |
| Kaliumoleat | 0,5 | 0,5 | 0,5 |
| Kaliumhydroxid | 0,09 | 0,09 | 0,09 |
| EDTA, Dinatriumsalz | 0,03 | 0,03 | 0,03 |
| 2-Octyldodecanol | 2,2 | 2,2 | 2,2 |
| Cetearylalkohol (C16-C18 Fettalkohole) | 13,2 | 13,2 | 13,2 |
| Ceteareth-20 | 3,3 | 3,3 | 3,3 |
| Titandioxid | 0,5 | 0,5 | 0,5 |
| Merquat Plus 3330 (Polyquaternium-39), Dimethyldiallylammoniumchlorid, Acrylamid, Acrylsäure, Terpolymer | 1,5 | 1,5 | 1,5 |
| Ammoniak (25 %ige wässrige Lösung | 6,0 | 6,0 | 6,0 |
| p-Toluylendiamin, Sulfat | 3,1 | 3,5 | 2,4 |
| Resorcin | -- | 1,0 | 0,2 |
| 2-Amino-3-hydroxypryridin | --- | --- | 0,8 |
| 2-Amino-4-(hydroxyethylamino)anisol, Sulfat | 1,0 | -- | 1,0 |
| m-Aminophenol | --- | 1,7 | 0,7 |
| Wasser | ad 100 | ad 100 | ad 100 |
| Malteserkreuze (multilamellare Vesikel) | ja | ja | ja |

| | FC4 | FC5 Vergleich | FC6 Vergleich |
|---|---|---|---|
| Carbomer (Polyacyrylsäure, Ammoniumsalz, Homo-Poylmer) | 0,2 | 0,2 | 0,2 |
| C16-C18-Fettalkoholsulfat, Natriumsalz | 0,7 | 0,7 | 0,7 |
| Natriumlaurethsulfat (C12-C14-Fettalkohol, 2-3 EO) | 1,3 | 1,3 | 1,3 |
| Kaliumoleat | 0,5 | 0,5 | 0,5 |
| Kaliumhydroxid | 0,09 | 0,09 | 0,09 |
| EDTA, Dinatriumsalz | 0,03 | 0,03 | 0,03 |
| 2-Octyldodecanol | 2,2 | 2,2 | 2,2 |
| Cetearylalkohol (C16-C18 Fettalkohole) | 13,2 | 13,2 | 13,2 |
| Ceteareth-20 | 3,3 | 3,3 | 3,3 |
| Titandioxid | 0,5 | 0,5 | 0,5 |
| Merquat Plus 3330 (Polyquaternium-39), Dimethyldiallylammoniumchlorid, Acrylamid, Acrylsäure, Terpolymer | 1,5 | 1,5 | 1,5 |
| Ammoniak (25 %ige wässrige Lösung | 6,0 | 6,0 | 6,0 |
| p-Toluylendiamin, Sulfat | 2,0 | 0,5 | --- |
| 2-(2-Hydroxyethyl)-p-phenylendiamin Sulfat | 1,1 | 2,3 | 4,1 |
| 1-Naphthol | 1,8 | 2,2 | 1,6 |
| 5-Amino-2-methylphenol | 0,1 | --- | 1,2 |
| Wasser | ad 100 | ad 100 | ad 100 |
| Malteserkreuze (multilamellare Vesikel) | ja | ja | ja |

| | FC7 | FC8 | FC9 |
|---|---|---|---|
| Carbomer (Polyacyrylsäure, Ammoniumsalz, Homo-Poylmer) | 0,2 | 0,2 | 0,2 |
| C16-C18-Fettalkoholsulfat, Natriumsalz | 0,7 | 0,7 | 0,7 |
| Natriumlaurethsulfat (C12-C14-Fettalkohol, 2-3 EO) | 1,3 | 1,3 | 1,3 |
| Kaliumoleat | 0,5 | 0,5 | 0,5 |
| Kaliumhydroxid | 0,09 | 0,09 | 0,09 |
| EDTA, Dinatriumsalz | 0,03 | 0,03 | 0,03 |
| 2-Octyldodecanol | 2,2 | 2,2 | 2,2 |
| Cetearylalkohol (C16-C18 Fettalkohole) | 13,2 | 13,2 | 13,2 |
| Ceteareth-20 | 3,3 | 3,3 | 3,3 |
| Titandioxid | 0,5 | 0,5 | 0,5 |
| Merquat Plus 3330 (Polyquaternium-39), Dimethyldiallylammoniumchlorid, Acrylamid, Acrylsäure, Terpolymer | 1,5 | 1,5 | 1,5 |
| Ammoniak (25 %ige wässrige Lösung | 6,0 | 6,0 | 6,0 |
| 4,5-Diamino-1-(2-hydroxyethyl)-1 H-pyrazol Sulfat | 3,4 | 3,0 | 2,9 |
| 3-Amino-6-chlor-2-methylphenol | 3,1 | 0,05 | -- |
| 5-Amino-2-methylphenol | ---- | 1,5 | 1,6 |
| 2-Methylresorcin | 0,1 | 2,4 | 0,5 |
| Wasser | ad 100 | ad 100 | ad 100 |
| Malteserkreuze (multilamellare Vesikel) | ja | ja | ja |

## Patentansprüche

1. Verfahren zur Herstellung eines Mittels (A) zur Farbveränderung von keratinischen Fasern, insbesondere menschlichen Haaren, welches in einem wässrigen Medium unilamellare Vesikel (ULV) und/oder multilamellare Vesikel (MLV) umfasst, wobei das Mittel enthält
(a1) mindestens einen C₈-C₃₀-Fettalkohol
(a2) mindestens einen ethoxylierten Fettalkohol der Formel (I), 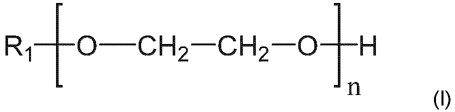 worin
R1 für eine lineare oder verzweigte, gesättigte oder ungesättigte C₈-C₃₀-Alkylgruppe steht und
n für eine ganze Zahl von 10 bis 100 steht,
(a3) mindestens ein anionisches Tensid der Formel (II) 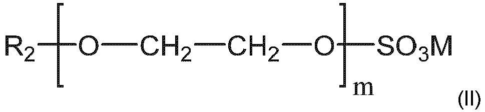 worin
R2 für eine lineare oder verzweigte, gesättigte oder ungesättigte C₈-C₃₀-Alkylgruppe steht, m für eine ganze Zahl von 0 bis 10 steht und
M für ein Wasserstoffatom, ein Äquivalent eines Natriumions, eines Kaliumions oder eines Ammoniumions (NH₄⁺) steht, und
(a4) ein oder mehrere farbverändernde Salze aus der Gruppe der Sulfate, der Hydrogensulfate, der Chloride und/oder der Bromide in einer Gesamtmenge von 0,9 bis 3,4 Gew.-%, wobei die Gesamtmenge auf das Gesamtgewicht des Mittels (A) bezogen ist,
**dadurch gekennzeichnet, dass** das Verfahren die folgenden Schritte umfasst
(I) Erhitzen eines Gemisches aus (a1) C₁₂-C₃₀-Fettalkohol, (a2) ethoxyliertem Fettalkohol der Formel (I), (a3) anionischem Tensid der Formel (II) und einer ersten Teilmenge Wasser auf eine Temperatur von 65 bis 85 °C in einem ersten Behältnis,
(II) Herstellung eines Gemisches aus einer zweiten Teilmenge Wasser und den farbverändernden Salzen aus der Gruppe der Sulfate, der Hydrogensulfate, der Chloride (a4) in einem zweiten Behältnis,
(III) Zugabe der in Schritt (II) erhaltenen Zubereitung zu der in Schritt (I) erhaltenen, auf 40 bis 60 °C temperierten Zubereitung unter konstantem Rühren mit einem Propeller-Rührwerk bei mindestens 500 Umdrehungen pro Minute,
(IV) Abkühlen des in Schritt (III) erhaltenen Gemisches unter ständigem Rühren auf 20 bis 40 °C.

2. Verfahren zur Herstellung eines Mittels (A) nach Anspruch 1, **dadurch gekennzeichnet, dass** das Mittel multilamellare Vesikel (MLV) umfasst

3. Verfahren zur Herstellung eines Mittels (A) nach einem der Ansprüche 1 bis 2, **dadurch gekennzeichnet, dass** das Mittel multilamellare Vesikel (MLV) mit einem mittleren Durchmesser von 0,02 bis 6,0 µm (Mikrometer), bevorzugt von 0,2 bis 2,0 µm (Mikrometer) umfasst.

4. Verfahren zur Herstellung eines Mittels (A) nach einem der Ansprüche 1 bis 3, **dadurch gekennzeichnet, dass** das Mittel - bezogen auf das Gesamtgewicht des Mittels (A) - (a1) einen oder mehrere lineare, gesättigte C₈-C₃₀-Fettalkohole in einer Gesamtmenge von 5,0 bis 25,0 Gew.%, bevorzugt von 7,0 bis 20,0 Gew.-%, weiter bevorzugt von 10,0 bis 18,0 Gew.-% und besonders bevorzugt von 12,0 bis 16,0 Gew.-% enthält.

5. Verfahren zur Herstellung eines Mittels (A) nach einem der Ansprüche 1 bis 4, **dadurch gekennzeichnet, dass** das Mittel - bezogen auf das Gesamtgewicht des Mittels (A) - (a1) einen oder mehrere verzweigte, gesättigte C₁₂-C₃₀ Fettalkohole in einer Gesamtmenge von 1,0 bis 9,0 Gew.-%, bevorzugt von 1,3 bis 7,0 Gew.-%, weiter bevorzugt von 1,6 bis 5,0 Gew.-% und besonders bevorzugt von 1,9 bis 3,0 Gew.-% enthält.

6. Verfahren zur Herstellung eines Mittels (A) nach einem der Ansprüche 1 bis 5, **dadurch gekennzeichnet, dass** das Mittel (a2) mindestens einen ethoxylierten Fettalkohol der Formel (I) enthält, worin
R1 für eine lineare oder verzweigte, gesättigte oder ungesättigte C₈-C₃₀-Alkylgruppe steht
und
n für eine ganze Zahl von 10 bis 30 steht.

7. Verfahren zur Herstellung eines Mittels (A) nach einem der Ansprüche 1 bis 6, **dadurch gekennzeichnet, dass** das Mittel - bezogen auf das Gesamtgewicht des Mittels (A) - (a2) einen oder mehrere ethoxylierte Fettalkohole der Formel (I) in einer Gesamtmenge von 0,5 bis 5,0 Gew.%, bevorzugt von 1,0 bis 4,5 Gew.-%, weiter bevorzugt von 1,5 bis 4,0 Gew.-% und besonders bevorzugt von 2,0 bis 3,5 Gew.-% enthält.

8. Verfahren zur Herstellung eines Mittels (A) nach einem der Ansprüche 1 bis 7, **dadurch gekennzeichnet, dass** das Mittel
| | |
|---|---|
| (a31) | mindestens ein erstes anionisches Tensid der Formel (II) enthält, worin |
| | R2 für eine lineare, gesättigte C₈-C₃₀-Alkylgruppe steht und m für die Zahl 0 steht, und |
| (a32) | mindestens ein zweites anionisches Tensid der Formel (II) enthält, worin R2 für eine lineare, gesättigte C₈-C₃₀-Alkylgruppe steht und m für eine ganze Zahl von 1 bis 5 steht. |

9. Verfahren zur Herstellung eines Mittels (A) nach einem der Ansprüche 1 bis 8, **dadurch gekennzeichnet, dass** das Mittel - bezogen auf das Gesamtgewicht des Mittels (A) - (a3) ein oder mehrere anionische Tenside der Formel (II) in einer Gesamtmenge von 0,5 bis 4,8 Gew.-% bevorzugt von 0,5 bis 4,2 Gew.-%, weiter bevorzugt von 0,5 bis 3,8 Gew.-% und besonders bevorzugt von 1,0 bis 3,0 Gew.-% enthält.

10. Verfahren zur Herstellung eines Mittels (A) nach einem der Ansprüche 1 bis 9, **dadurch gekennzeichnet, dass** das Mittel (a4) ein oder mehrere farbverändernde Salze aus der Gruppe aus Phenylendiamin Sulfat, Phenylendiamin Monohydrochlorid, Phenylendiamin Dihydrochlorid, p-Toluylendiamin Sulfat, p-Toluylendiamin Monohydrochlorid, p-Toluylendiamin Dihydrochlorid, 2-(2-Hydroxyethyl)-p-phenylendiamin Sulfat, 2-(2-Hydroxyethyl)-p-phenylendiamin Monohydrochlorid, 2-(2-Hydroxyethyl)-p-phenylendiamin Dihydrochlorid, N,N-Bis-(2-hydroxyethyl)-p-phenylendiamin Sulfat, N,N-Bis-(2-hydroxyethyl)-p-phenylendiamin Monohydrochlorid, N,N-Bis-(2-hydroxyethyl)-p-phenylendiamin Dihydrochlorid, 2-Methoxymethyl-p-phenylendiamin Sulfat, 2-Methoxymethyl-p-phenylendiamin Monohydrochlorid, 2-Methoxymethyl-p-phenylendiamin Dihydrochlorid, p-Aminophenol Hydrogensulfat, p-Aminophenol Monohydrochlorid, 4-Amino-3-methylphenol Hydrogensulfat, 4-Amino-3-methylphenol Chlorid, 2,4,5,6-Tetraaminopyrimidin Monosulfat, 2,4,5,6-Tetraaminopyrimidin Disulfat, 2,4,5,6-Tetraaminopyrimidin Monohydrochlorid, 2,4,5,6-Tetraaminopyrimidin Dihydrochlorid, 2,4,5,6-Tetraaminopyrimidin Trihydrochlorid, 2,4,5,6-Tetraaminopyrimidin Tetrahydrochlorid, 4-Hydroxy-2,5,6-triaminopyrimidin Sulfat, 4-Hydroxy-2,5,6-triaminopyrimidin Monohydrochlorid, 4-Hydroxy-2,5,6-triaminopyrimidin Diydrochlorid, 4-Hydroxy-2,5,6-triaminopyrimidin Trihydrochlorid, 4,5-Diamino-1-(2-hydroxyethyl)-1H-pyrazol Sulfat, 4,5-Diamino-1-(2-hydroxyethyl)-1H-pyrazol Monohydrochlorid und/oder 4,5-Diamino-1-(2-hydroxyethyl)-1H-pyrazol Dihydrochlorid enthält.

11. Verfahren zur Herstellung eines Mittels (A) nach einem der Ansprüche 1 bis 10, **dadurch gekennzeichnet, dass** das Mittel - bezogen auf das Gesamtgewicht des Mittels (A) - (a4) ein oder mehrere farbverändernde farbverändernde Salze aus der Gruppe der Sulfate, der Hydrogensulfate, der Chloride und/oder der Bromide in einer Gesamtmenge von 2,1 bis 3,4 Gew.-% enthält.

12. Verfahren zur Herstellung eines Mittels (A) nach einem der Ansprüche 1 bis 11, **dadurch gekennzeichnet, dass** das Mittel - bezogen auf das Gesamtgewicht des Mittels (A) - phosphorhaltige Verbindungen in einer Gesamtmenge von weniger als 0,1 Gew.-%, bevorzugt von weniger als 0,05 Gew.-% enthält.

13. Verfahren zur Herstellung eines Mittels (A) nach einem der Ansprüche 1 bis 12, **dadurch gekennzeichnet, dass** das Mittel
(a5) mindestens ein C₈-C₃₀-Fettsäuremonoglycerid, ein C₈-C₃₀-Fettsäurediglycerid und/oder ein C₈-C₃₀-Fettsäuretriglycerid enthält.

14. Verfahren zur Herstellung eines Mittels (A) nach einem der Ansprüche 1 bis 13, **dadurch gekennzeichnet, dass** das Mittel - bezogen auf das Gesamtgewicht des Mittels (A) - (a5) ein oder mehrere C₈-C₃₀-Fettsäuremonoglyceride in einer Gesamtmenge von 1,0 bis 6,0 Gew.-%, bevorzugt von 1,5 bis 5,5 Gew.-%, weiter bevorzugt von 2,0 bis 5,0 Gew.-% und ganz besonders bevorzugt von 2,5 bis 4,5 Gew.-% enthält.

15. Verfahren nach einem der Ansprüche 1 bis 14, **gekennzeichnet durch** die
(III) Zugabe der in Schritt (II) erhaltenen Zubereitung zu der in Schritt (I) erhaltenen, auf 40 bis 60 °C temperierten Zubereitung unter konstantem Rühren mit einem Propeller-Rührwerk bei mindestens 800 Umdrehungen pro Minute.

## Claims

1. A method for preparing an agent (A) for changing the color of keratin fibers, in particular human hair, which comprises unilamellar vesicles (ULV) and/or multilamellar vesicles (MLV) in an aqueous medium, the agent containing
(a1) at least one C₈-C₃₀ fatty alcohol
(a2) at least one ethoxylated fatty alcohol of formula (I) 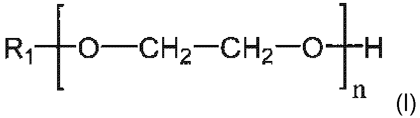 where
R1 represents a linear or branched, saturated or unsaturated C₈-C₃₀ alkyl group and
n represents an integer from 10 to 100,
(a3) at least one anionic surfactant of formula (II) 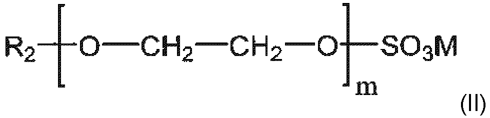 where
R2 represents a linear or branched, saturated or unsaturated C₈-C₃₀ alkyl group,
m represents an integer from 0 to 10 and
M represents a hydrogen atom, an equivalent of a sodium ion, potassium ion or ammonium ion (NH4⁺), and
(a4) one or more color-changing salts from the group of sulfates, hydrogen sulfates, chlorides and/or bromides in a total amount of 0.9 to 3.4 wt.%, the total amount being based on the total weight of the agent (A),
**characterized in that** the method comprises the following steps
(I) heating a mixture consisting of (a1) C₁₂-C₃₀ fatty alcohol, (a2) ethoxylated fatty alcohol of formula (I), (a3) anionic surfactant of formula (II) and a first partial amount of water to a temperature of 65 to 85 °C in a first container,
(II) preparing a mixture consisting of a second partial amount of water and (a4) the color-changing salts from the group of sulfates, hydrogen sulfates and chlorides in a second container,
(III) adding the preparation obtained in step (II) to the preparation obtained in step (I) and temperature-controlled to 40 to 60 °C, with constant stirring using a propeller agitator at at least 500 revolutions per minute,
(IV) cooling the mixture obtained in step (III) to 20 to 40 °C with constant stirring.

2. The method for preparing an agent (A) according to claim 1, **characterized in that** the agent comprises multilamellar vesicles (MLV).

3. The method for preparing an agent (A) according to one of claims 1 to 2, **characterized in that** the agent comprises multilamellar vesicles (MLV) having an average diameter of 0.02 to 6.0 µm (micrometers), preferably 0.2 to 2.0 µm (micrometers).

4. The method for preparing an agent (A) according to one of claims 1 to 3, **characterized in that** the agent contains (a1) one or more linear, saturated C₈-C₃₀ fatty alcohols in a total amount of 5.0 to 25.0 wt.%, preferably 7.0 to 20.0 wt.%, more preferably 10.0 to 18.0 wt.% and particularly preferably 12.0 to 16.0 wt.%, based on the total weight of the agent (A).

5. The method for preparing an agent (A) according to one of claims 1 to 4, **characterized in that** the agent contains (a1) one or more branched, saturated C₁₂-C₃₀ fatty alcohols in a total amount of 1.0 to 9.0 wt.%, preferably 1.3 to 7.0 wt.%, more preferably 1.6 to 5.0 wt.% and particularly preferably 1.9 to 3.0 wt.%, based on the total weight of the agent (A).

6. The method for preparing an agent (A) according to one of claims 1 to 5, **characterized in that** the agent contains (a2) at least one ethoxylated fatty alcohol of formula (I), where R1 represents a linear or branched, saturated or unsaturated C₈-C₃₀ alkyl group and n represents an integer from 10 to 30.

7. The method for preparing an agent (A) according to one of claims 1 to 6, **characterized in that** the agent contains (a2) one or more ethoxylated fatty alcohols of formula (I) in a total amount of 0.5 to 5.0 wt.%, preferably 1.0 to 4.5 wt.%, more preferably 1.5 to 4.0 wt.% and particularly preferably 2.0 to 3.5 wt.%, based on the total weight of the agent (A).

8. The method for preparing an agent (A) according to one of claims 1 to 7, **characterized in that** the agent contains
| | |
|---|---|
| (a31) | at least one first anionic surfactant of formula (II), where R2 represents a linear, saturated C₈-C₃₀ alkyl group and m represents the number 0, and |
| (a32) | at least one second anionic surfactant of formula (II), where R2 represents a linear, saturated C₈-C₃₀ alkyl group and |
| m | represents an integer from 1 to 5. |

9. The method for preparing an agent (A) according to one of claims 1 to 8, **characterized in that** the agent contains (a3) one or more anionic surfactants of formula (II) in a total amount of 0.5 to 4.8 wt.%, preferably 0.5 to 4.2 wt.%, more preferably 0.5 to 3.8 wt.%, and particularly preferably 1.0 to 3.0 wt.%, based on the total weight of the agent (A).

10. The method for preparing an agent (A) according to one of claims 1 to 9, **characterized in that** the agent contains (a4) one or more color-changing salts from the group of phenylenediamine sulfate, phenylenediamine monohydrochloride, phenylenediamine dihydrochloride, p-toluenediamine sulfate, p-toluenediamine monohydrochloride, p-toluenediamine dihydrochloride, 2-(2-hydroxyethyl)-p-phenylenediamine sulfate, 2-(2-hydroxyethyl)-p-phenylenediamine monohydrochloride, 2-(2-hydroxyethyl)-p-phenylenediamine dihydrochloride, N,N-bis-(2-hydroxyethyl)-p-phenylenediamine sulfate, N,N-bis-(2-hydroxyethyl)-p-phenylenediamine monohydrochloride, N,N-bis-(2-hydroxyethyl)-p-phenylenediamine dihydrochloride, 2-methoxymethyl-p-phenylenediamine sulfate, 2-methoxymethyl-p-phenylenediamine monohydrochloride, 2-methoxymethyl-p-phenylenediamine dihydrochloride, p-aminophenol hydrogen sulfate, p-aminophenol monohydrochloride, 4-amino-3-methylphenol hydrogen sulfate, 4-amino-3-methylphenol chloride, 2,4,5,6-tetraaminopyrimidine monosulfate, 2,4,5,6-tetraaminopyrimidine disulfate, 2,4,5,6-tetraaminopyrimidine monohydrochloride, 2,4,5,6-tetraaminopyrimidine dihydrochloride, 2,4,5,6-tetraaminopyrimidine trihydrochloride, 2,4,5,6-tetraaminopyrimidine tetrahydrochloride, 4-hydroxy-2,5,6-triaminopyrimidine sulfate, 4-hydroxy-2,5,6-triaminopyrimidine monohydrochloride, 4-hydroxy-2,5,6-triaminopyrimidine diydrochloride, 4-hydroxy-2,5,6-triaminopyrimidine trihydrochloride, 4,5-diamino-1-(2-hydroxyethyl)-1H-pyrazole sulfate, 4,5-diamino-1-(2-hydroxyethyl)-1H-pyrazole monohydrochloride and/or 4,5-diamino-1-(2-hydroxyethyl)-1H-pyrazole dihydrochloride.

11. The method for preparing an agent (A) according to one of claims 1 to 10, **characterized in that** the agent contains (a4) one or more color-changing salts from the group of sulfates, hydrogen sulfates, chlorides and/or bromides in a total amount of 2.1 to 3.4 wt.%, based on the total weight of the agent (A).

12. The method for preparing an agent (A) according to one of claims 1 to 11, **characterized in that** the agent contains phosphorus-containing compounds in a total amount of less than 0.1 wt.%, preferably less than 0.05 wt.%, based on the total weight of the agent (A).

13. The method for preparing an agent (A) according to one of claims 1 to 12, **characterized in that** the agent contains
(a5) at least one C₈-C₃₀ fatty acid monoglyceride, C₈-C₃₀ fatty acid diglyceride and/or C₈-C₃₀ fatty acid triglyceride.

14. The method for preparing an agent (A) according to one of claims 1 to 13, **characterized in that** the agent contains (a5) one or more C₈-C₃₀ fatty acid monoglycerides in a total amount of 1.0 to 6.0 wt.%, preferably 1.5 to 5.5 wt.%, more preferably 2.0 to 5.0 wt.% and very particularly preferably 2.5 to 4.5 wt.%, based on the total weight of the agent (A).

15. The method according to one of claims 1 to 14, **characterized by** the following step
(III) adding the preparation obtained in step (II) to the preparation obtained in step (I) and temperature-controlled to 40 to 60 °C, with constant stirring using a propeller agitator at at least 800 revolutions per minute.

## Revendications

1. Procédé de production d'un agent (A) destiné à modifier la couleur de fibres kératiniques, en particulier de cheveux humains, qui comprend dans un milieu aqueux des vésicules unilamellaires (ULV) et/ou des vésicules multilamellaires (MLV), l'agent contenant
(a1) au moins un alcool gras en C₈-C₃₀
(a2) au moins un alcool gras éthoxylé de formule (I), 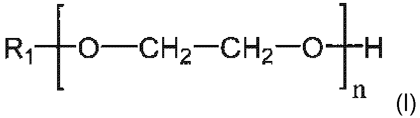 dans laquelle
R1 représente un groupe alkyle en C₈-C₃₀, linéaire ou ramifié, saturé ou insaturé, et
n représente un nombre entier de 10 à 100,
(a3) au moins un tensioactif anionique de formule (II) 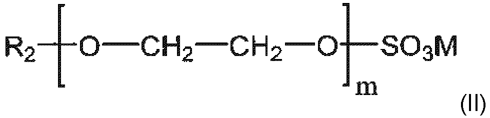 dans laquelle
R2 représente un groupe alkyle en C₈-C₃₀, linéaire ou ramifié, saturé ou insaturé, m représente un nombre entier de 0 à 10, et
M représente un atome d'hydrogène, un équivalent d'un ion sodium, d'un ion potassium ou d'un ion ammonium (NH₄⁺), et
(a4) un ou plusieurs sels modificateurs de couleur du groupe des sulfates, des hydrogénosulfates, des chlorures et/ou des bromures en une quantité totale de 0,9 à 3,4 % en poids, la quantité totale étant basée sur le poids total de l'agent (A), **caractérisé en ce que** le procédé comprend les étapes suivantes
(I) le chauffage d'un mélange de (a1) l'alcool gras en C₁₂-C₃₀, de (a2) l'alcool gras éthoxylé de formule (I), du (a3) tensioactif anionique de formule (II) et d'une première quantité partielle d'eau à une température de 65 à 85 °C dans un premier récipient,
(II) la préparation d'un mélange d'une seconde quantité partielle d'eau et des sels modificateurs de couleur du groupe des sulfates, des hydrogénosulfates et des chlorures (a4) dans un second récipient,
(III) l'ajout de la préparation obtenue à l'étape (II) à la préparation obtenue à l'étape (I), thermorégulée à 40 à 60 °C, sous agitation constante à l'aide d'un agitateur à hélice à au moins 500 tours par minute,
(IV) le refroidissement du mélange obtenu à l'étape (III) à 20 à 40 °C sous agitation constante.

2. Procédé de production d'un agent (A) selon la revendication 1, **caractérisé en ce que** l'agent comprend des vésicules multilamellaires (MLV).

3. Procédé de production d'un agent (A) selon l'une des revendications 1 à 2, **caractérisé en ce que** l'agent comprend des vésicules multilamellaires (MLV) d'un diamètre moyen de 0,02 à 6,0 µm (micromètres), de préférence de 0,2 à 2,0 µm (micromètres).

4. Procédé de production d'un agent (A) selon l'une des revendications 1 à 3, **caractérisé en ce qu'**il contient - par rapport au poids total de l'agent (A) - (a1) un ou plusieurs alcools gras en C₈-C₃₀ linéaires saturés en une quantité totale de 5,0 à 25,0 % en poids, de préférence de 7,0 à 20,0 % en poids, de manière davantage préférée de 10,0 à 18,0 % en poids et de manière particulièrement préférée de 12,0 à 16,0 % en poids.

5. Procédé de production d'un agent (A) selon l'une des revendications 1 à 4, **caractérisé en ce qu'**il contient - par rapport au poids total de l'agent (A) - (a1) un ou plusieurs alcools gras en C₁₂-C₃₀ ramifiés saturés en une quantité totale de 1,0 à 9,0 % en poids, de préférence de 1,3 à 7,0 % en poids, de manière davantage préférée de 1,6 à 5,0 % en poids et de manière particulièrement préférée de 1,9 à 3,0 % en poids.

6. Procédé de préparation d'un agent (A) selon l'une des revendications 1 à 5, **caractérisé en ce que** l'agent (a2) contient au moins un alcool gras éthoxylé de formule (I), dans laquelle
R1 représente un groupe alkyle en C₈-C₃₀, linéaire ou ramifié, saturé ou insaturé, et
n représente un nombre entier de 10 à 30.

7. Procédé de production d'un agent (A) selon l'une des revendications 1 à 6, **caractérisé en ce qu'**il contient - par rapport au poids total de l'agent (A) - (a2) un ou plusieurs alcools gras éthoxylés de formule (I) en une quantité totale de 0,5 à 5,0 % en poids, de préférence de 1,0 à 4,5 % en poids, de manière davantage préférée de 1,5 à 4,0 % en poids et de manière particulièrement préférée de 2,0 à 3,5 % en poids.

8. Procédé de production d'un agent (A) selon l'une des revendications 1 à 7, **caractérisé en ce que** l'agent
(a31) contient au moins un premier tensioactif anionique de formule (II), dans laquelle
R2 représente un groupe alkyle linéaire saturé en C₈-C₃₀, et
m représente le nombre 0, et
(a32) contient au moins un second tensioactif anionique de formule (II), dans laquelle
R2 représente un groupe alkyle linéaire saturé en C₈-C₃₀, et
m représente un nombre entier de 1 à 5.

9. Procédé de production d'un agent (A) selon l'une des revendications 1 à 8, **caractérisé en ce qu'**il contient - par rapport au poids total de l'agent (A) - (a3) un ou plusieurs tensioactifs anioniques de formule (II) en une quantité totale de 0,5 à 4,8 % en poids, de préférence de 0,5 à 4,2 % en poids, de manière davantage préférée de 0,5 à 3,8 % en poids et de manière particulièrement préférée de 1,0 à 3,0 % en poids.

10. Procédé de production d'un agent (A) selon l'une des revendications 1 à 9, **caractérisé en ce que** l'agent (a4) contient un ou plusieurs sels modificateurs de couleur du groupe constitué par le sulfate de phénylènediamine, le monochlorhydrate de phénylènediamine, le dichlorhydrate de phénylènediamine, le sulfate de p-tolylènediamine, le monochlorhydrate de p-tolylènediamine, le dichlorhydrate de p-tolylènediamine, le sulfate de 2-(2-hydroxyéthyl)-p-phénylènediamine, le monochlorhydrate de 2-(2-hydroxyéthyl)-p-phénylènediamine, le dichlorhydrate de 2-(2-hydroxyéthyl)-p-phénylènediamine, le sulfate de N,N-bis-(2-hydroxyéthyl)-p-phénylènediamine, le monochlorhydrate de N,N-bis-(2-hydroxyéthyl)-p-phénylènediamine, le dichlorhydrate de N,N-bis-(2-hydroxyéthyl)-p-phénylènediamine, le sulfate de 2-méthoxyméthyl-p-phénylènediamine, le monochlorhydrate de 2-méthoxyméthyl-p-phénylènediamine, le dichlorhydrate de 2-méthoxyméthyl-p-phénylènediamine, l'hydrogénosulfate de p-aminophénol, le monochlorhydrate de p-aminophénol, l'hydrogénosulfate de 4-amino-3-méthylphénol, le chlorure de 4-amino-3-méthylphénol, le monosulfate de 2,4,5,6-tétraaminopyrimidine, le disulfate de 2,4,5,6-tétraaminopyrimidine, le monochlorhydrate de 2,4,5,6-tétraaminopyrimidine, le dichlorhydrate de 2,4,5,6-tétraaminopyrimidine, le trichlorhydrate de 2,4,5,6-tétraaminopyrimidine, le tétrachlorhydrate de 2,4,5,6-tétraaminopyrimidine, le sulfate de 4-hydroxy-2,5,6-triaminopyrimidine, le monochlorhydrate de 4-hydroxy-2,5,6-triaminopyrimidine, le dichlorhydrate de 4-hydroxy-2,5,6-triaminopyrimidine, le trichlorhydrate de 4-hydroxy-2,5,6-triaminopyrimidine, le sulfate de 4,5-diamino-1-(2-hydroxyéthyl)-1H-pyrazole, le monochlorhydrate de 4,5-diamino-1-(2-hydroxyéthyl)-1H-pyrazole et/ou le dichlorhydrate de 4,5-diamino-1-(2-hydroxyéthyl)-1H-pyrazole.

11. Procédé de production d'un agent (A) selon l'une des revendications 1 à 10, **caractérisé en ce que** l'agent contient - par rapport au poids total de l'agent (A) - (a4) un ou plusieurs sels modificateurs de couleur du groupe des sulfates, des hydrogénosulfates, des chlorures et/ou des bromures en une quantité totale de 2,1 à 3,4 % en poids.

12. Procédé de production d'un agent (A) selon l'une des revendications 1 à 11, **caractérisé en ce que** l'agent contient - par rapport au poids total de l'agent (A) - des composés contenant du phosphore en une quantité totale inférieure à 0,1 % en poids, de préférence inférieure à 0,05 % en poids.

13. Procédé de production d'un agent (A) selon l'une des revendications 1 à 12, **caractérisé en ce que** l'agent
(a5) contient au moins un monoglycéride d'acide gras en C₈-C₃₀, un diglycéride d'acide gras en C₈-C₃₀ et/ou un triglycéride d'acide gras en C₈-C₃₀.

14. Procédé de production d'un agent (A) selon l'une des revendications 1 à 13, **caractérisé en ce qu'**il contient - par rapport au poids total de l'agent (A) - (a5) un ou plusieurs monoglycérides d'alcools gras en C₈-C₃₀ en une quantité totale de 1,0 à 6,0 % en poids, de préférence de 1,5 à 5,5 % en poids, de manière davantage préférée de 2,0 à 5,0 % en poids et de manière particulièrement préférée de 2,5 à 4,5 % en poids.

15. Procédé selon l'une des revendications 1 à 14, **caractérisé par**
(III) l'ajout de la préparation obtenue à l'étape (II) à la préparation obtenue à l'étape (I), thermorégulée à 40 à 60 °C, sous agitation constante à l'aide d'un agitateur à hélice à au moins 800 tours par minute.
